# EUROPEAN PATENT SPECIFICATION

(11) **EP 4 540 223 B1**
(45) Date of publication and mention of the grant of the patent: **04.11.2026**
(21) Application number: 23731702.9
(22) Date of filing: 13.06.2023
(51) Int. Cl.: C07C 229/52, C07C 227/42, A61Q 1/00, A61K 8/00

(54) **METHOD FOR OBTAINING CRYSTALLINE DIETHYLAMINO HYDROXYBENZOYL HEXYL BENZOATE**
VERFAHREN ZUR HERSTELLUNG VON KRISTALLINEM DIETHYLAMINOHYDROXYBENZOYLHEXYLBENZOAT
PROCÉDÉ D'OBTENTION DE DIÉTHYLAMINO HYDROXYBENZOYL HEXYL BENZOATE CRISTALLIN

(30) Priority: 17.06.2022 EP 22179682
(43) Date of publication of application: 23.04.2025
(73) Proprietor: BASF SE, 67056 Ludwigshafen am Rhein (DE)
(72) Inventor: SCHEIN-ALBRECHT, Karin, 67056 Ludwigshafen am Rhein (DE); KLEINMANN, Eva, 67056 Ludwigshafen am Rhein (DE); KRONEMAYER, Helmut, 67056 Ludwigshafen am Rhein (DE); BINDER, Axel, 67056 Ludwigshafen am Rhein (DE); EHLIS, Thomas, 79100 Freiburg (DE)
(74) Representative: BASF IP Association
(86) International application number: PCT/EP2023/065783
(87) International publication number: WO 2023/242181

(56) References cited:
- WO-A1-2021/170695

## Description

### Field of invention

The presently claimed invention relates to methods for obtaining crystalline diethylamino hydroxybenzoyl hexyl benzoate. Further, the presently claimed invention relates to crystalline diethylamino hydroxybenzoyl hexyl benzoate obtained by the methods of the invention, purified diethylamino hydroxybenzoyl hexyl benzoate obtained by the methods of the invention and to a cosmetic composition comprising purified diethylamino hydroxybenzoyl hexyl benzoate according to the invention.

### Background of the invention

UV radiation causes harmful effects on the human skin such as sunburn, phototoxic and photo allergenic reactions, acceleration of skin aging and increase in the risk of skin cancer. To protect the human skin from UV radiation, sunscreen or cosmetic compositions comprising UV filters (also referred to as UV absorbers) are used.

In general, UV light can be divided into UV-A radiation (320 - 400 nm) and UV-B radiation (280 - 320 nm). Since 2006, the EU commission has recommended that all sunscreen or cosmetic compositions should have an UV-A protection factor, which is at least one third of the labelled sun protection factor (SPF), wherein the sun protection factor refers mainly to the UV-B protection.

2-(4'-Diethylamino-2'-hydroxybenzoyl)benzoic acid hexyl ester (DHHB) is an effective UV-A filter. It is represented by formula (I).

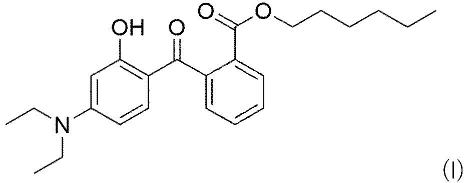

2-(4'-Diethylamino-2'-hydroxybenzoyl)benzoic acid hexyl ester (I) preparation is known, e.g. from DE 10011317, EP2155660, and WO 2003097578.

However, the product obtained from known processes are associated with several disadvantages.

Generally, 2-(4'-diethylamino-2'-hydroxybenzoyl)benzoic acid hexyl ester (I) is obtained as a melt after the completion of synthesis process and is stored in the same form. Crystal growth occurs in the melt after about six weeks at room temperature. At the time of use, the user is required to heat the entire pack to a temperature above the melting point of (I) to be able to remove product from the storage containers.

The product contains phthalic acid dihexyl ester (II) (also known as PSDHE, or dihexylphthalate, or di-n-hexylphthalate) as an impurity. The PSDHE content should be as low as possible, since this substance is toxic; it can damage fertility and may have harmful effects on the unborn child. According to the harmonized classification and labelling (ATP05), the allowable amount of PSDHE (II) in the final product is up to 150 ppm.

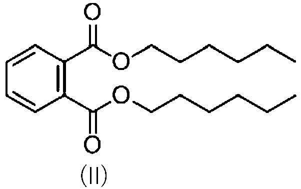

Further, the product obtained by known processes contains rhodamine type dyes (e.g. [9-(2-carboxyphenyl)-6-diethylamino-3-xanthenylidene]-diethylammonium salts) (III) and corresponding esters (IV) as impurities, which lead to an undesired discoloration of the final product.

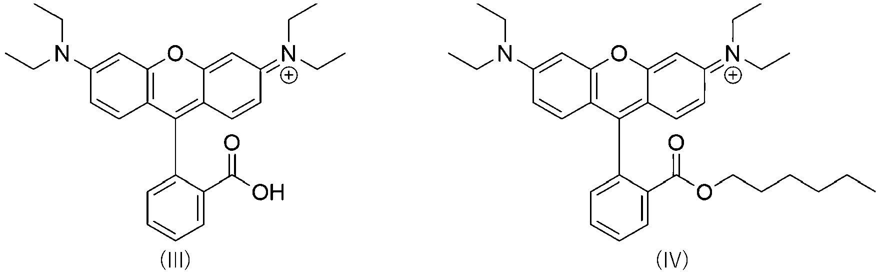

Further, the product obtained by known processes might contain the potential intermediate 2-(4'-diethylamino-2'-hydroxybenzoyl)benzoic acid (V).

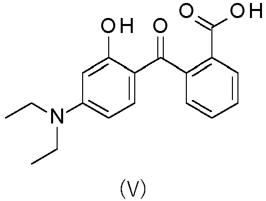

WO 2021/170695 A1 describes a process for isolating 2-(4'-Diethylamino-2'-hydroxybenzoyl)benzoic acid hexyl ester (I) in good purity. The process described therein is directed to isolation processes in solvents with a boiling point of at most 110° C, limiting the scope of the process to short-chain hydrocarbon solvents. Additionally, WO 2021/1700695 A1 requires a solvent switch from the solvent used for the preparation 2-(4'-Diethylamino-2'-hydroxybenzoyl)benzoic acid hexyl ester (I) to the solvent used in the esterification of benzoic acid derivative (V), potentially impacting process efficiency.

Due to these challenges, there is an ongoing need for a fast and efficient method for obtaining crystalline 2-(4'-diethylamino-2'-hydroxybenzoyl)benzoic acid hexyl ester (I) as well as obtaining crystalline (I) having a low amount of (II), and low amounts of (III), (IV) and (V).

Accordingly, it is an object of the present invention to provide a method for obtaining crystalline 2-(4'-diethylamino-2'-hydroxybenzoyl)benzoic acid hexyl ester (I). Further, it is an object to provide a method to obtain crystalline 2-(4'-diethylamino-2'-hydroxybenzoyl)benzoic acid hexyl ester (I) having a low amount of phthalic acid dihexyl ester (II). Furthermore, it is desired that the method provides crystalline 2-(4'-diethylamino-2'-hydroxybenzoyl)benzoic acid hexyl ester having low amounts of rhodamine based impurities (III) and (IV) and a low amount of 2-(4'-diethylamino-2'-hydroxybenzoyl)benzoic acid (V).

### Summary of the invention

Surprisingly, it is found that at least one of the above objects is achieved by the method according to the presently claimed invention.

In a first aspect, the present invention is directed to a method for obtaining crystalline diethylamino hydroxybenzoyl hexyl benzoate (I) comprising steps (a) to (d),
(a) providing a feed comprising diethylamino hydroxybenzoyl hexyl benzoate (I) and at least a first crystallization solvent, and heating the feed to obtain a solution, wherein the diethylamino hydroxybenzoyl hexyl benzoate (I) has i) a phthalic acid dihexyl ester (PSDHE) (II) content of up to 100000 ppm;
   based on the weight of diethylamino hydroxybenzoyl hexyl benzoate (I); and
   wherein the amount of the diethylamino hydroxybenzoyl hexyl benzoate (I) in the feed is in the range of 15.0 to 65.0 wt% based on the total weight of the feed;
(b) cooling the solution obtained in step (a) to obtain a supersaturated solution of diethylamino hydroxybenzoyl hexyl benzoate (I);
(c) seeding the supersaturated solution obtained in step (b) with seed crystals of diethylamino hydroxybenzoyl hexyl benzoate (I);
   wherein the amount of the seed crystals is in the range from 0.01 to 15.0 wt% based on the weight of diethylamino hydroxybenzoyl hexyl benzoate (I) in the solution; and
(d) cooling the solution obtained in step (c) to a temperature in the range of -20 to 17 ° C to obtain a suspension comprising crystalline diethylamino hydroxybenzoyl hexyl benzoate (I); followed by separating crystalline diethylamino hydroxybenzoyl hexyl benzoate (I) and washing the separated crystalline diethylamino hydroxybenzoyl hexyl benzoate (I) with a washing solvent at least a first time and a second time;
   and
(e) optionally recrystallizing the diethylamino hydroxybenzoyl hexyl benzoate (I) obtained in step (d) involving mixing the diethylamino hydroxybenzoyl hexyl benzoate (I) obtained in step (d) with at least a second crystallization solvent to obtain a feed, heating the feed to obtain a solution, and using the method steps (b) to (d);
wherein the quantity of washing solvent is in each of the at least first time and second time washing in at least one of step (d) of the crystallization or step (d) of the recrystallization (e) at least 63 wt.%, based on the weight of the diethylamino hydroxybenzoyl hexyl benzoate (I) in the corresponding solution in step (b), wherein the first crystallization solvent comprises at least one C₄-C₈ alcohol.

In a second aspect, the present invention is directed to a method for obtaining crystalline diethylamino hydroxybenzoyl hexyl benzoate (I) comprising steps (a) to (d),
(a) providing a feed comprising diethylamino hydroxybenzoyl hexyl benzoate (I) and at least a first crystallization solvent, and heating the feed to obtain a solution, wherein the diethylamino hydroxybenzoyl hexyl benzoate (I) has i) a phthalic acid dihexyl ester (PSDHE) (II) content of up to 100000 ppm;
   based on the weight of diethylamino hydroxybenzoyl hexyl benzoate (I); and
   wherein the amount of the diethylamino hydroxybenzoyl hexyl benzoate (I) in the feed is in the range of 15.0 to 65.0 wt% based on the total weight of the feed;
(b) cooling the solution obtained in step (a) to obtain a supersaturated solution of diethylamino hydroxybenzoyl hexyl benzoate (I);
(c) seeding the supersaturated solution obtained in step (b) with seed crystals of diethylamino hydroxybenzoyl hexyl benzoate (I);
   wherein the amount of the seed crystals is in the range from 0.01 to 15.0 wt% based on the weight of diethylamino hydroxybenzoyl hexyl benzoate (I) in the solution; and
(d) cooling the solution obtained in step (c) to a temperature in the range of -20 to 17 ° C to obtain a suspension comprising crystalline diethylamino hydroxybenzoyl hexyl benzoate (I); followed by separating crystalline diethylamino hydroxybenzoyl hexyl benzoate (I) and washing the separated crystalline diethylamino hydroxybenzoyl hexyl benzoate (I) with a washing solvent;
   and
(e) optionally recrystallizing the diethylamino hydroxybenzoyl hexyl benzoate (I) obtained in step (d) involving mixing the diethylamino hydroxybenzoyl hexyl benzoate (I) obtained in step (d) with the at least a second crystallization solvent to obtain a feed, heating the feed to obtain a solution, and using the method steps (b) to (d);
wherein the quantity of washing solvent is in at least one of step (d) of the crystallization or step (d) of the recrystallization (e) at least 130 wt.%, based on the weight of the diethylamino hydroxybenzoyl hexyl benzoate (I) in the corresponding solution in step (b), wherein the first crystallization solvent comprises at least one C₄-C₈ alcohol.

In a third aspect, the present invention is directed to a crystalline diethylamino hydroxybenzoyl hexyl benzoate (I) obtained by one of the methods according to the invention having
i) a phthalic acid dihexyl ester (PSDHE) (II) content of less than 250 ppm;
ii) a rhodamine (III) content of less than 150 ppm; and
a rhodamine hexyl ester (IV) content of less than 150 ppm;each based on the weight of the crystalline diethylamino hydroxybenzoyl hexyl benzoate (I).

In a fourth aspect, the present invention is directed to a purified diethylamino hydroxybenzoyl hexyl benzoate (I) obtained by one of the methods according to the invention having
i) a phthalic acid dihexyl ester (PSDHE) (II) content of less than 250 ppm;
ii) a rhodamine (III) content of less than 10 ppm; and
a rhodamine hexyl ester (IV) content of less than 10 ppm; each based on the weight of the purified diethylamino hydroxybenzoyl hexyl benzoate (I)

In a fifth aspect, the present invention is directed to a cosmetic composition comprising the purified diethylamino hydroxybenzoyl hexyl benzoate (I) of the invention.

### Detailed Description

Before the present compositions and formulations of the presently claimed invention are described, it is to be understood that this invention is not limited to particular compositions and formulations described, since such compositions and formulation may, of course, vary. It is also to be understood that the terminology used herein is not intended to be limiting, since the scope of the presently claimed invention will be limited only by the appended claims.

If hereinafter a group is defined to comprise at least a certain number of embodiments, this is meant to also encompass a group which preferably consists of these embodiments only. Furthermore, the terms 'first', 'second', 'third' or 'a', 'b', 'c', etc. and the like in the description and in the claims, are used for distinguishing between similar elements and not necessarily for describing a sequential or chronological order. It is to be understood that the terms so used are interchangeable under appropriate circumstances and that the embodiments of the presently claimed invention described herein are capable of operation in other sequences than described or illustrated herein. In case the terms 'first', 'second', 'third' or '(A)', '(B)' and '(C)' or '(a)', '(b)', '(c)', '(d)', 'i', 'ii' etc. relate to steps of a method or use or assay there is no time or time interval coherence between the steps, that is, the steps may be carried out simultaneously or there may be time intervals of seconds, minutes, hours, days, weeks, months or even years between such steps, unless otherwise indicated in the application as set forth herein above or below.

Furthermore, the ranges defined throughout the specification include the end values as well i.e. a range of 1 to 10 implies that both 1 and 10 are included in the range. For the avoidance of doubt, applicant shall be entitled to any equivalents according to applicable law.

In the following passages, different aspects of the presently claimed invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to 'one embodiment' or 'an embodiment' means that a particular feature, structure or characteristic described in connection with the embodiment is included in at least one embodiment of the presently claimed invention. Thus, appearances of the phrases 'in one embodiment' or 'in an embodiment' in various places throughout this specification are not necessarily all referring to the same embodiment.

Furthermore, the particular features, structures or characteristics may be combined in any suitable manner, as would be apparent to a person skilled in the art from this disclosure, in one or more embodiments. Furthermore, while some embodiments described herein include some, but not other features included in other embodiments, combinations of features of different embodiments are meant to be within the scope of the presently claimed invention, and form different embodiments, as would be understood by those in the art. For example, in the appended claims, any of the claimed embodiments can be used in any combination.

The term "ultraviolet filter" or "UV filter" as used herein refers to organic or inorganic compounds, which can absorb and/or reflect UV radiation caused by sunlight. UV filter can be classified based on their UV protection curve as UV-A, UV-B or broadband filters. In the context of the present application, broadband filters may be listed as UV-A filters, as they also provide UV-A protection. In other words, preferred UV-A filters also include broadband filters.

The definition of "broadband" protection (also referred to as broad-spectrum or broad protection) is based on the "critical wavelength". For broadband coverage, UV-B and UV-A protection must be provided. According to the US requirements, a critical wavelength of at least 370 nm is required for achieving broad spectrum protection. Furthermore, it is recommended by the European Commission that all sunscreen or cosmetic compositions should have an UVA protection factor, which is at least one third of the labelled sun protection factor (SPF), e.g. if the sunscreen composition has an SPF of 30, the UVA protection factor has to be at least 10.

The term "sunscreen composition" or "sunscreen" or "skin-care product" refers to any topical product, which reflects and/or absorbs certain parts of UV radiation. Thus, the term "sunscreen composition" is to be understood as not only including sunscreen compositions, but also any cosmetic compositions that provide UV protection. The term "topical product" refers to a product that is applied to the skin and can refer, e.g., to sprays, lotions, creams, oils, foams, powders, or gels. According to the present invention the sunscreen composition may comprise one or more active agents, e.g., organic UV filters, as well as other ingredients or additives, e.g., emulsifiers, emollients, viscosity regulators, stabilizers, preservatives, or fragrances.

The terms "diethylamino hydroxybenzoyl hexyl benzoate" and "DHHB" and "compound (I)" or "(I)" are used synonymously in the present invention. They all refer to the compound of formula (I) depicted above.

The term "crystallization solvent" refers to a solvent used for the crystallization of a material. The crystallization solvent provides for the material to be crystallized a certain threshold solubility at certain temperatures or concentrations. Therefore, the material can be dissolved in the crystallization solvent at certain temperatures and/or concentrations but may start crystallizing once the temperatures is decreased or the concentration of the material in the crystallization solvent is increased.

The term "washing solvent" refers to a solvent used for washing a solid material. Generally, the washing solvent should not dissolve the solid material during washing, so that only impurities and byproducts are removed from the solid material.

The term "solvent" is to be understood as a substance that can dissolve a solute, resulting in a solution.

The terms "n-hexanol" and "1-hexanol" are used synonymously in the present invention.

The terms "C₁-C₈ alcohols" or "C₄-C₈ alcohols" and similar refer to compounds comprising at least one hydroxyl functional group "-OH" and a carbon chain that comprises the amount of carbon atoms in the suffix, i.e. C₃ comprises three carbon atoms. The alcohol can be a primary (RCH₂OH), a secondary (R₂CHOH) or a tertiary (R₃COH) alcohol. Furthermore, the carbon chain of the alcohol can be linear as in n-Propanol or n-Hexanol, it can be branched as in *iso-*Butanol, it can be cyclic as in Cyclohexanol and it can be saturated or unsaturated.

The alcohols can have one (monohydric alcohol) or more (polyhydric alcohol) hydroxyl functional groups. Furthermore, the alcohols can have other functional groups at different positions of the molecule. The person skilled in the art is aware of other functional groups that can be present in C₁-C₈ or C₄-C₈ alcohols.

Surprisingly, it is found that the method according to the presently claimed invention provides crystalline 2-(4'-diethylamino-2'-hydroxybenzoyl)benzoic acid hexyl ester (I) having a low PSDHE (II) content as well as low content of rhodamine based impurities (III) and (IV). The method of the presently claimed invention is fast and efficient.

The method of the presently claimed invention improves the quality and purity of crystalline (I) and reduces the time required for the crystallization by selection of appropriate crystallization process parameters such as crystallization solvent, degree of supersaturation, the amount of seed crystals of (I), the temperature of seeding, the process temperature, the quantity of washing solvent as well as time of each process step.

According to a first aspect of the invention, the present invention is directed to a method for obtaining crystalline diethylamino hydroxybenzoyl hexyl benzoate (I) comprising steps (a) to (d),
(a) providing a feed comprising diethylamino hydroxybenzoyl hexyl benzoate (I) and at least a first crystallization solvent, and heating the feed to obtain a solution, wherein the diethylamino hydroxybenzoyl hexyl benzoate (I) has i) a phthalic acid dihexyl ester (PSDHE) (II) content of up to 100000 ppm;
   based on the weight of diethylamino hydroxybenzoyl hexyl benzoate (I); and
   wherein the amount of the diethylamino hydroxybenzoyl hexyl benzoate (I) in the feed is in the range of 15.0 to 65.0 wt% based on the total weight of the feed;
(b) cooling the solution obtained in step (a) to obtain a supersaturated solution of diethylamino hydroxybenzoyl hexyl benzoate (I);
(c) seeding the supersaturated solution obtained in step (b) with seed crystals of diethylamino hydroxybenzoyl hexyl benzoate (I);
   wherein the amount of the seed crystals is in the range from 0.01 to 15.0 wt% based on the weight of diethylamino hydroxybenzoyl hexyl benzoate (I) in the solution; and
(d) cooling the solution obtained in step (c) to a temperature in the range of -20 to 17 ° C to obtain a suspension comprising crystalline diethylamino hydroxybenzoyl hexyl benzoate (I); followed by separating crystalline diethylamino hydroxybenzoyl hexyl benzoate (I) and washing the separated crystalline diethylamino hydroxybenzoyl hexyl benzoate (I) with a washing solvent at least a first time and a second time;
   and
(e) optionally recrystallizing the diethylamino hydroxybenzoyl hexyl benzoate (I) obtained in step (d) involving mixing the diethylamino hydroxybenzoyl hexyl benzoate (I) obtained in step (d) with at least a second crystallization solvent to obtain a feed, heating the feed to obtain a solution, and using the method steps (b) to (d);
wherein the quantity of washing solvent is in each of the at least first time and second time washing in at least one of step (d) of the crystallization or step (d) of the recrystallization (e) at least 63 wt.%, based on the weight of the diethylamino hydroxybenzoyl hexyl benzoate (I) in the corresponding solution in step (b), wherein the first crystallization solvent comprises at least one C₄-C₈ alcohol.

Therefore, according to one aspect of the invention, the washing of the separated crystalline DHHB occurs with a washing solvent at least a first time and a second time, wherein the quantity of washing solvent is in each of the at least first time and second time washing in at least one of step (d) of the crystallization or step (d) of the recrystallization (e) at least 63 wt.%, based on the weight of the diethylamino hydroxybenzoyl hexyl benzoate (I) in the corresponding solution in step (b). It is possible to add further washing steps in this approach by washing the separated crystalline DHHB a third time, a fourth time, a fifth time, a sixth time and so on.

According to a preferred embodiment of the first aspect of the invention, the quantity of washing solvent is in each of the at least first time and second time washing in at least one of step (d) of the crystallization or step (d) of the recrystallization (e) at least 65 wt.%, preferably at least 68 wt.%, more preferred at least 70 wt.% and most preferred at least 74 wt.%, based on the weight of the diethylamino hydroxybenzoyl hexyl benzoate (I) in the corresponding solution in step (b).

According to a second aspect of the invention, the present invention is directed to a method for obtaining crystalline diethylamino hydroxybenzoyl hexyl benzoate (I) comprising steps (a) to (d),
(a) providing a feed comprising diethylamino hydroxybenzoyl hexyl benzoate (I) and at least a first crystallization solvent, and heating the feed to obtain a solution, wherein the diethylamino hydroxybenzoyl hexyl benzoate (I) has
   i) a phthalic acid dihexyl ester (PSDHE) (II) content of up to 100000 ppm;
      based on the weight of diethylamino hydroxybenzoyl hexyl benzoate (I); and
      wherein the amount of the diethylamino hydroxybenzoyl hexyl benzoate (I) in the feed is in the range of 15.0 to 65.0 wt% based on the total weight of the feed;
(b) cooling the solution obtained in step (a) to obtain a supersaturated solution of diethylamino hydroxybenzoyl hexyl benzoate (I);
(c) seeding the supersaturated solution obtained in step (b) with seed crystals of diethylamino hydroxybenzoyl hexyl benzoate (I);
   wherein the amount of the seed crystals is in the range from 0.01 to 15.0 wt% based on the weight of diethylamino hydroxybenzoyl hexyl benzoate (I) in the solution; and
(d) cooling the solution obtained in step (c) to a temperature in the range of -20 to 17 ° C to obtain a suspension comprising crystalline diethylamino hydroxybenzoyl hexyl benzoate (I); followed by separating crystalline diethylamino hydroxybenzoyl hexyl benzoate (I) and washing the separated crystalline diethylamino hydroxybenzoyl hexyl benzoate (I) with a washing solvent;
   and
(e) optionally recrystallizing the diethylamino hydroxybenzoyl hexyl benzoate (I) obtained in step (d) involving mixing the diethylamino hydroxybenzoyl hexyl benzoate (I) obtained in step (d) with the at least a second crystallization solvent to obtain a feed, heating the feed to obtain a solution, and using the method steps (b) to (d);
wherein the quantity of washing solvent is in at least one of step (d) of the crystallization or step (d) of the recrystallization (e) at least 130 wt.%, based on the weight of the diethylamino hydroxybenzoyl hexyl benzoate (I) in the corresponding solution in step (b), wherein the first crystallization solvent comprises at least one C₄-C₈ alcohol.

In the second aspect of the inventive, the separated crystalline DHHB is washed with a washing solvent, wherein the quantity of washing solvent is in at least one of step (d) of the crystallization or step (d) of the recrystallization (e) at least 130 wt.%, based on the weight of the diethylamino hydroxybenzoyl hexyl benzoate (I) in the corresponding solution in step (b). In this aspect of the invention, the mandatory total quantity of washing solvent used for the washing step is defined. It is possible to conduct the washing with the entire quantity of washing solvent by washing the crystalline DHHB one time. However, it is equally possible to spread the total quantity of washing solvent used over several times of washing such as washing the separated crystalline DHHB at least a first time and a second time or washing the separated crystalline DHHB at least a first time, a second time and a third time or washing the separated crystalline DHHB at least a first time, a second time, a third time and a fourth time. In general, there is no limitation to the times of washing the separated crystalline DHHB. It has been found that two to six times washing, in particular two to three times washing the separated crystalline DHHB allow for an optimal balance between process efficiency and product purity.

The following embodiments are directed to the methods of the first and the second aspect of the invention.

In case the washing of the separated crystalline DHHB is performed by washing the crystalline DHHB at least a first time and a second time, the quantity of washing solvent can be the same in both the first and the second time washing. However, it is also possible that the quantity of washing solvent used in the first time and the second time washing are different. For example, the quantity of washing solvent used in the first time washing can be greater than the quantity of washing solvent used in the second time washing. Alternatively, the quantity of washing solvent used in the second time washing can be greater than the quantity of washing solvent used in the first time washing.

In case the crystalline DHHB is washed a third, fourth, fifth, sixth and so on time, the quantity for each of these times of washing the DHHB can be chosen independently from the quantity of washing solvent used in the first and second time washing.

According to a preferred embodiment of the invention, the total quantity of washing solvent used for washing the separated crystalline DHHB is at least 132 wt.%, more preferred at least 135 wt.%, even more preferred at least 140 wt.% or at least 145 wt.%, particularly preferred at least 150 wt.% and most preferred at least 160 wt.%, based on the weight of the diethylamino hydroxybenzoyl hexyl benzoate (I) in the corresponding solution in step (b).

According to a preferred embodiment of the invention, the diethylamino hydroxybenzoyl hexyl benzoate (I) in step (a) has
i) a rhodamine (III) content of less than 15000 ppm; and/or
ii) a rhodamine hexyl ester (IV) content of less than 15000 ppm;
each based on the weight of diethylamino hydroxybenzoyl hexyl benzoate (I). DHHB with such an impurity profile regarding rhodamine (III) and rhodamine hexyl ester (IV) can be completely and effectively purified with the crystallization methods of the invention.

According to another preferred embodiment of the invention, the diethylamino hydroxybenzoyl hexyl benzoate (I) in step (a) has a 2-(4'-diethylamino-2'-hydroxybenzoyl)benzoic acid (V) content of less than 5000 ppm, based on the weight of diethylamino hydroxybenzoyl hexyl benzoate (I).

According to a preferred embodiment of the invention, the solution in step (b) is cooled to a temperature such that the ratio of c : c* of the concentration c of dissolved diethylamino hydroxybenzoyl hexyl benzoate (I) to the equilibrium solubility c* of diethylamino hydroxybenzoyl hexyl benzoate (I) at the temperature is in the range from 1.1:1.0 to 9.0:1.0. Further preferred, the ratio of c:c* is in the range of 1.5:1.0 to 7.0:1.0 and even more preferred in the range from 2.0:1.0 to 6.0:1.0. Temperatures at a ratio of c:c* of 3.8:1.0, 4.0:1.0 and 4.1:1.0 are most preferred. It was discovered that at a temperature with such a specific ratio of c : c*, the crystallization process finds an optimal balance between rate of crystallization and purity of the product obtained.

According to another preferred embodiment, the diethylamino hydroxybenzoyl hexyl benzoate (I) (DHHB) in step (a) has a phthalic acid dihexyl ester (PSDHE) (II) content of 10 to 70000 ppm, in particular from 500 to 50000 ppm or from 5000 to 40000 or from 7000 to 30000 ppm, based on the weight of diethylamino hydroxybenzoyl hexyl benzoate (I). It was found that DHHB with such a PSDHE content can be particularly quickly and effectively purified with the inventive methods.

The inventive methods use a first crystallization solvent in step (a). According to a preferred embodiment, the first crystallization solvent comprises or consists of at least one selected from the group consisting of C₁-C₈ alcohols. It was discovered that C₁-C₈ alcohols are excellent crystallization solvents for DHHB in the present method. They only show a low solubility for DHHB at low temperatures while allowing for a good solubility at higher temperatures, thus allowing for the effective crystallization of DHHB when cooling the supersaturated solution of DHHB in step (b).

It was found that C₄-C₈ alcohols are particularly preferred as the first crystallization solvent. Therefore, according to one particularly preferred embodiment, the first crystallization solvent comprises or consists of C₄-C₈ alcohols.

In case a recrystallization step (e) is performed, a second crystallization solvent is used. In this case, it is preferred if the second crystallization solvent comprises or consists of at least one selected from the group consisting of C₁-C₈ alcohols. It was discovered that C₁-C₈ alcohols are excellent crystallization solvents for DHHB in the present method. They only show a low solubility for DHHB at low temperatures while allowing for a good solubility at higher temperatures, thus allowing for the effective crystallization of DHHB when cooling the supersaturated solution of DHHB in step (b). It was found that C₄-C₈ alcohols are particularly preferred as the seconds crystallization solvent. Therefore, according to one particularly preferred embodiment, the second crystallization solvent comprises or consists of C₄-C₈ alcohols.

In case a recrystallization step (e) is performed, the first and the second crystallization solvent are preferably both C₁-C₈ alcohols. According to one preferred embodiment, the first and the second crystallization solvent can be the same, for example both the first and the second crystallization solvent can be 1-hexanol. Such an approach allows for an effective and resource conserving approach. However, it is also possible according to the present invention that the first and the second crystallization solvent are different, for example that the first crystallization solvent is 1-hexanol and the second crystallization solvent is iso-Propanol. This approach allows for an optimal fine-tuning between the crystallization and the recrystallization step to allow for a particularly pure crystalline DHHB product.

**It** was discovered that in case a recrystallization step (e) takes place it is preferable that the first crystallization solvent is selected from the group consisting of C₄-C₈ alcohols and the second crystallization solvent is selected from the group consisting of C₁-C₈ alcohols. It is particularly preferred that the first crystallization solvent comprises or consists of 1-hexanol and the second crystallization solvent comprises or consists of an alcohol selected from methanol, ethanol, *n*-propanol and *iso*-propanol. With such a combination of first and second crystallization solvent, all relevant side products and impurities can be removed particularly effectively with the inventive methods.

According to one particularly preferred embodiment, the first crystallization solvent comprises or consists of 1-hexanol.

If the last step of the synthesis of diethylamino hydroxybenzoyl hexyl benzoate (I) is an esterification of diethylamino hydroxybenzoyl benzoic acid with 1-hexanol, the product obtained from the esterification step comprises a mixture of diethylamino hydroxybenzoyl hexyl benzoate (I) and 1-hexanol. In this case, it is a preferred embodiment that the feed exiting the esterification reaction is directly used in the crystallization methods of the invention.

In a preferred embodiment, the product obtained from the esterification comprising diethylamino hydroxybenzoyl hexyl benzoate (I) and 1-hexanol is directly used as a feed for the crystallization.

According to another preferred embodiment of the invention, the second crystallization solvent comprises or consists of 1-hexanol.

In step (d) of the methods of the present invention, the separated crystalline DHHB is washed with a washing solvent. According to a preferred embodiment of the invention, the washing solvent comprises or consists of an alcohol, in particular a C₁-C₈ alcohol. It is particularly preferred in the present invention that the washing solvent comprises an alcohol selected from methanol, ethanol, iso-propanol, n-propanol, iso-butanol, tert-butanol, n-butanol, n-pentanol, sec-pentanol, cyclopentanol, n-hexanol, sec-hexanol, cyclohexanol, n-heptanol, sec-heptanol, n-octanol, sec-octanol. It was found that alcohols and the alcohols listed in particular are well suited as washing solvents in the inventive methods. They remove impurities effectively from crystalline DHHB. It is particularly preferred that the washing solvent is 1-hexanol.

In case a recrystallization step (e) is performed, the washing solvent used in the recrystallization can be the same as in the crystallization. However, it is also possible that the washing solvent used in the recrystallization step (e) differs from the washing solvent used in the crystallization. For example, it is possible that the washing solvent used in the crystallization comprises or consists of 1-hexanol and the washing solvent used in the recrystallization comprises or consists of iso-propanol. Consequently, the first crystallization solvent, the second crystallization solvent, the washing solvent in the crystallization and the washing solvent in the recrystallization can all be selected from the herein identified preferred solvents independently from one another.

It is preferred that the washing of the separated crystalline diethylamino hydroxybenzoyl hexyl benzoate (I) is carried with a washing solvent having a temperature within ± 5 ° C of the temperature to which the solution is cooled in step (d). In this way, it is ensured that no DHHB is dissolved by the washing solvent and only impurities are removed.

In another preferred embodiment, the first washing solvent and/or the second washing solvent is cooled to a temperature in the range of -5 to 5 ° C. In this way, it is ensured that no DHHB is dissolved by the washing solvent and only impurities are removed.

The crystalline DHHB of step d is separated in any technically sensible way. The skilled person is aware of different techniques of filtering crystalline materials in a chemical process, for example by using a nutsche filter or an agitated nutsche filter or a centrifuge, for example a pusher-, a decanter- a sieve decanter or a peeler centrifuge or a drum filter, for example a rotary- or a vacuum drum filter or a belt filter and the like. For example, filtration can be done by suction or by pressure.

In general, the washing of the separated crystalline DHHB with the washing solvent can be conducted in any technically sensible way. The skilled person is aware of different techniques of washing crystalline materials in a chemical process. However, it is particularly preferred that the washing is performed by displacement wash or by reslurry/suspension wash.

In a displacement wash, the washing solvent is passed through the filtered crystalline DHHB and the first or second crystallization solvent. Displacement washing can be done by suction, pressure or by gravitational forces.

In contrast, in a reslurry/suspension wash, a suspension of the separated crystalline DHHB in the washing solvent is first prepared and preferably agitated, for example for a period of 2 to 50 minutes, more preferably for a period of 10 to 30 minutes. Next, the suspension is filtered to obtain crystalline DHHB. The passage through the filter may again be performed by suction, pressure or by gravitational forces. For a reslurry/suspension wash an agitated nutsche filter is particularly preferred.

According to a particularly preferred embodiment of the present invention, the first and/or second crystallization solvent and/or the washing solvent is/are recycled after use. It was found that the first and/or second crystallization solvent as well as the washing solvent can be effectively reused via recycling in the present invention. This results in an ecological and resource-saving process.

In case the first and/or second crystallization solvent and/or the washing solvent is/are recycled after use, it is preferred according to one embodiment that the first and/or second crystallization solvent and/or washing solvent is/are purified for recycling after use. This is preferred in case the starting material DHHB has a high impurity content. However, according to another embodiment, it is also preferred that the first and/or second crystallization solvent and/or washing solvent is/are not purified for recycling after use. This allows for a more efficient and less complicated recycling of the solvents. In addition, it saves further resources and reduces costs.

In a preferred embodiment, in step (a) the feed is heated to a temperature in the range of 25 to 80 ° C. In a more preferred embodiment, in step (a) the feed is heated to a temperature in the range of 35 to 80 ° C, most preferably in the range of 40 to 60 ° C and in particular preferably in the range of 43 to 52 ° C. It was found that these are the ideal temperatures to obtain a complete dissolution of DHHB in the crystallization solvent without wasting energy.

In a preferred embodiment, in step (b) the solution is cooled to a temperature in the range of by 18 to 35 ° C, more preferably in the range of 18 to 32 ° C and most preferably in the range of 23 to 30 ° C. It was discovered through extensive experimentation that this is the ideal temperature range to obtain the supersaturated solution of DHHB which forms the basis for the crystallization of DHHB.

The crystallization of (I) on its own is very slow due to the high metastable character of liquid (I). Seeding of the supersaturated solution is an important step during the crystallization of the presently claimed invention. Due to seeding, the crystallization starts early as compared to that without seed crystals. Hence, the overall process of the presently claimed invention provides crystalline (I) faster as compared to a process that does not involve the step of seeding.

In addition to the other process parameters, carrying out the seeding using a right amount of crystals of (I) followed by the stirring at the temperature of seeding is important for the success of the presently claimed invention.

The quality of crystalline (I) obtained during the crystallization depends upon the temperature at which seeding is carried out as well as upon the amount of seed crystals.

In a preferred embodiment, in step (c) the seeding is carried out at a temperature in the range of 18 to 35 ° C, more preferably in the range of 18 to 32 ° C and most preferably in the range of 23 to 30 ° C.

In a preferred embodiment, the seed crystals of diethylamino hydroxybenzoyl hexyl benzoate (I) are employed in step (c) in at least one form selected from a solid crystalline mass and a suspension in a solvent.

In a preferred embodiment, the seed crystals of diethylamino hydroxybenzoyl hexyl benzoate (I) are employed in step (c) in the form of a suspension in the crystallization solvent in step (a).

In a more preferred embodiment, the seed crystals of diethylamino hydroxybenzoyl hexyl benzoate (I) are employed in step (c) in the form of a suspension in 1-hexanol.

In a preferred embodiment, the amount of the seed crystals is in the range of 0.01 to 15.0 wt%, more preferably in the range of 0.5 to 10.0 wt%, even more preferably in the range of 1.0 to 5.0 wt% and most preferably in the range of 1.5 to 4.5 wt%, based on the weight of diethylamino hydroxybenzoyl hexyl benzoate (I). This amount of seed crystals has been found to be particularly effective in promoting rapid crystallization.

It is further preferred that the supersaturated solution comprising seed crystals is agitated for up to 8 hours, in particular 20 minutes to 7 hours, after seeding the supersaturated solution obtained in step (b) with seed crystals of diethylamino hydroxybenzoyl hexyl benzoate (I) and before cooling in step (d). It was discovered that agitating the supersaturated solution of DHHB after introducing seed crystals of DHHB results in a good size distribution of the crystals and ensures rapid crystal formation. The agitation rate is chosen such that it is suited for crystal formation, for example that the agitation rate is high enough to allow a good distribution and flow of dissolved particles while having no detrimental impact on forming crystals.

In a preferred embodiment, step (d) involves cooling the solution obtained in step (c) to -10 to 15 ° C; more preferably to -5 to 10 ° C and most preferably to 0 to 5 ° C. In this way, crystallization of DHHB present in the supersaturated solution of step (b) is achieved in an energy-efficient way with high yield.

In a preferred embodiment, step (d) involves cooling the solution obtained in step (c) at a cooling rate of 1 to 15 K/h, more preferably at 3 to 10 K/h and most preferably at 4.5 to 8 K/h. These cooling rates have been found to be particularly beneficial for the crystallization process of DHHB.

Different cooling methods can be employed for the cooling in step (d). The skilled person is aware of multiple different methods of cooling. Preferably, the solution is cooled in one of the following ways: in a linear manner, with a piecewise linear cooling profile, with a parabolic cooling profile with increasing cooling rates, with a dynamic cooling profile to maintain a relatively constant supersaturation in the crystallizer or with vacuum cooling method.

According to a preferred embodiment of the invention, the suspension obtained in step (d) is agitated at the cooling temperature of step (d) for up to 12 hours, preferably for up to 2 hours and most preferred for up to one hour before separation of the crystalline DHHB.

In a preferred embodiment, in step (d) the separation of crystals is carried out by filtration while maintaining the temperature of the solution within ± 5 ° C of the temperature to which the solution is cooled in step (d). In this way, it is ensured that there is no dissolution of DHHB during this step.

It is preferred that the filter cake, that is the solid component that remains in the filter after filtration of the suspension, is dried after filtration. This is preferably done by suction or by passing through a drying gas, for example air or nitrogen, in particular nitrogen. In production processes, it is preferred to dry the filter cake by passing nitrogen gas through the filter cake. The filter cake is preferably dried for a period of up to 5 hours, more preferably of up to 1 hours and most preferred of from 5 to 30 minutes.

In a preferred embodiment, the method of the presently claimed invention further comprises the following steps,
i. dissolving the crystalline diethylamino hydroxybenzoyl hexyl benzoate (I) obtained in step (d) or step (e) in a purification solvent;
ii. contacting the solution obtained in step (i) with an adsorbent; and
iii. removal of the adsorbent and the purification solvent from the solution obtained in step (ii) to obtain purified diethylamino hydroxybenzoyl hexyl benzoate (I).

In a preferred embodiment, the purification solvent is selected from toluene, cyclohexane, and combinations thereof.

In a preferred embodiment, the adsorbent is selected from silica, charcoal and combinations thereof.

In a preferred embodiment, the purified diethylamino hydroxybenzoyl hexyl benzoate is subsequently solidified, in particular pelletized. Methods of solidifying the purified diethylamino hydroxybenzoyl hexyl benzoate are known to the skilled person. Melt crystallization at specific shear rates with or without seeding is a preferred way of solidifying DHHB. According to a particularly preferred embodiment, the purified diethylamino hydroxybenzoyl hexyl benzoate is solidified according to at least one of the solidification methods described in WO 2008/135360 A1, in PCT application PCT/EP2022/055088 and in PCT application PCT/EP2022/055089. The solidified DHHB is preferably further processed into pourable/flowable particles, pastilles or flakes. Methods of preparing such particles, pastilles or flakes from a solidified material are known to the skilled person in the art.

The crystallization and agitation of materials according to the present invention can be performed in any suitable crystallization and stirrer equipment. The skilled person is aware of common process equipment for crystallization and agitation of materials. In a preferred embodiment, the crystallization is carried out in a crystallizer selected from a stirred vessel with baffles, force circulation crystallizer, draft tube crystallizer, draft tube baffled crystallizer, and an Oslo type crystallizer. Any type of stirrer can be used. Preferred stirrers are for example a single or multi-stage inclined or non-inclined cross blade stirrer, an impeller stirrer or a propeller stirrer. Particularly preferred is an anchor stirrer or an inclined anchor stirrer.

The method of the presently claimed invention provides crystalline diethylamino hydroxybenzoyl hexyl benzoate (I) with a yield of 75.0 to 99.5 %, more preferably 90.0 to 99.0 % based on the total of (I) in the feed.

Another aspect of the invention is the crystalline diethylamino hydroxybenzoyl hexyl benzoate (I) obtained by the inventive methods. The crystalline diethylamino hydroxybenzoyl hexyl benzoate (I) according to the invention has
i) a phthalic acid dihexyl ester (PSDHE) (II) content of less than 250 ppm;
ii) a rhodamine (III) content of less than 150 ppm; and
iii) a rhodamine hexyl ester (IV) content of less than 150 ppm;
each based on the weight of the crystalline diethylamino hydroxybenzoyl hexyl benzoate (I).

In a preferred embodiment of the invention, the crystalline diethylamino hydroxybenzoyl hexyl benzoate (I) has a PSDHE (II) content of less than 120 ppm, preferably less than 60 ppm, more preferred less than 20 ppm, even more preferred less than 5 ppm and most preferred less than 1 ppm, based on the weight of the crystalline diethylamino hydroxybenzoyl hexyl benzoate (I).

In a preferred embodiment of the invention, the crystalline diethylamino hydroxybenzoyl hexyl benzoate (I) has a rhodamine (III) content of less than 80 ppm, preferably less than 40 ppm, more preferred less than 20 ppm, even more preferred less than 10 ppm and most preferred less than 5 ppm, based on the weight of the crystalline diethylamino hydroxybenzoyl hexyl benzoate (I).

In a preferred embodiment of the invention, the crystalline diethylamino hydroxybenzoyl hexyl benzoate (I) has a rhodamine hexyl ester (IV) content of less than 120 ppm, preferably less than 100 ppm, more preferred less than 50 ppm, even more preferred less than 10 ppm and most preferred less than 5 ppm, based on the weight of the crystalline diethylamino hydroxybenzoyl hexyl benzoate (I).

According to one embodiment of the invention, the crystalline diethylamino hydroxybenzoyl hexyl benzoate (I) has a 2-(4'-diethylamino-2'-hydroxybenzoyl)benzoic acid (V) content of less than 250 ppm, based on the weight of the crystalline diethylamino hydroxybenzoyl hexyl benzoate (I).

In a preferred embodiment of the invention, the crystalline diethylamino hydroxybenzoyl hexyl benzoate (I) has a 2-(4'-diethylamino-2'-hydroxybenzoyl)benzoic acid (V) content of less than 100 ppm, preferably less than 60 ppm, more preferred less than 15 ppm, even more preferred less than 5 ppm and most preferred less than 1 ppm, based on the weight of the crystalline diethylamino hydroxybenzoyl hexyl benzoate (I).

Certain combinations of by-products in the crystalline diethylamino hydroxybenzoyl hexyl benzoate (I) obtained by the inventive methods are particularly preferable. In particular, it is preferred that the inventive crystalline diethylamino hydroxybenzoyl hexyl benzoate (I) has a PSDHE (II) content of less than 120 ppm, a rhodamine (III) content of less than 150 ppm and a rhodamine hexyl ester (IV) content of less than 150 ppm; more preferred a PSDHE (II) content of less than 90 ppm, a rhodamine (III) content of less than 80 ppm and a rhodamine hexyl ester (IV) content of less than 120 ppm; even more preferred a PSDHE (II) content of less than 5 ppm, a rhodamine (III) content of less than 10 ppm and a rhodamine hexyl ester (IV) content of less than 10 ppm and most preferred a PSDHE (II) content of less than 1 ppm, a rhodamine (III) content of less than 5 ppm and a rhodamine hexyl ester (IV) content of less than 5 ppm; each based on the weight of the crystalline diethylamino hydroxybenzoyl hexyl benzoate (I).

Certain other combinations of by-products in the crystalline diethylamino hydroxybenzoyl hexyl benzoate (I) obtained by the inventive methods are also particularly preferable. In particular, it is preferred that the inventive crystalline diethylamino hydroxybenzoyl hexyl benzoate (I) has a PSDHE (II) content of less than 120 ppm, a rhodamine (III) content of less than 150 ppm, a rhodamine hexyl ester (IV) content of less than 150 ppm, and a 2-(4'-diethylamino-2'-hydroxybenzoyl)benzoic acid (V) content of less than 100 ppm; more preferred a PSDHE (II) content of less than 90 ppm, a rhodamine (III) content of less than 80 ppm, a rhodamine hexyl ester (IV) content of less than 120 ppm and a 2-(4'-diethylamino-2'-hydroxybenzoyl)benzoic acid (V) content of less than 60 ppm; even more preferred a PSDHE (II) content of less than 5 ppm, a rhodamine (III) content of less than 10 ppm, a rhodamine hexyl ester (IV) content of less than 10 ppm and a 2-(4'-diethylamino-2'-hydroxybenzoyl)benzoic acid (V) content of less than 10 ppm; and most preferred a PSDHE (II) content of less than 1 ppm, a rhodamine (III) content of less than 5 ppm, a rhodamine hexyl ester (IV) content of less than 5 ppm and a 2-(4'-diethylamino-2'-hydroxybenzoyl)benzoic acid (V) content of less than 1 ppm; each based on the weight of the crystalline diethylamino hydroxybenzoyl hexyl benzoate (I).

Crystalline DHHB with such a high purity and low amounts of problematic by-products was not accessible via previous crystallization methods.

Another aspect of the invention relates to the purified diethylamino hydroxybenzoyl hexyl benzoate (I) having
i) a phthalic acid dihexyl ester (PSDHE) (II) content of less than 250 ppm;
ii) a rhodamine (III) content of less than 10 ppm; and
iii) a rhodamine hexyl ester (IV) content of less than 10 ppm;
each based on the weight of the purified diethylamino hydroxybenzoyl hexyl benzoate (I). Purified DHHB is obtained by the inventive method when the crystalline DHHB is dissolved in a purification solvent and contacted with an adsorbent which is subsequently removed.

According to a preferred embodiment of the invention, the purified DHHB of the invention is solid. Particularly preferred the purified DHHB is in form of pourable/flowable particles, pastilles or flakes.

In a preferred embodiment of the invention, the purified diethylamino hydroxybenzoyl hexyl benzoate (I) has a PSDHE (II) content of less than 120 ppm, preferably less than 60 ppm, more preferred less than 20 ppm, even more preferred less than 5 ppm and most preferred less than 1 ppm, based on the weight of the purified diethylamino hydroxybenzoyl hexyl benzoate (I).

In a preferred embodiment of the invention, the purified diethylamino hydroxybenzoyl hexyl benzoate (I) has a rhodamine (III) content of less than 8 ppm, preferably less than 5 ppm most preferred less than 1 ppm, based on the weight of the purified diethylamino hydroxybenzoyl hexyl benzoate (I).

In a preferred embodiment of the invention, the purified diethylamino hydroxybenzoyl hexyl benzoate (I) has a rhodamine hexyl ester (IV) content of less than 8 ppm, preferably less than 5 ppm and most preferred less than 1 ppm, based on the weight of the purified diethylamino hydroxybenzoyl hexyl benzoate (I).

According to one embodiment of the invention, the purified diethylamino hydroxybenzoyl hexyl benzoate (I) has a 2-(4'-diethylamino-2'-hydroxybenzoyl)benzoic acid (V) content of less than 250 ppm, based on the weight of the purified diethylamino hydroxybenzoyl hexyl benzoate (I).

In a preferred embodiment of the invention, the purified diethylamino hydroxybenzoyl hexyl benzoate (I) has a 2-(4'-diethylamino-2'-hydroxybenzoyl)benzoic acid (V) content of less than 100 ppm, preferably less than 60 ppm, more preferred less than 15 ppm, even more preferred less than 5 ppm and most preferred less than 1 ppm, based on the weight of the crystalline diethylamino hydroxybenzoyl hexyl benzoate (I).

Certain combinations of by-products in the purified diethylamino hydroxybenzoyl hexyl benzoate (I) obtained by the inventive methods are particularly preferable. In particular, it is preferred that the inventive purified diethylamino hydroxybenzoyl hexyl benzoate (I) has a PSDHE (II) content of less than 120 ppm, a rhodamine (III) content of less than 10 ppm and a rhodamine hexyl ester (IV) content of less than 10 ppm; more preferred a PSDHE (II) content of less than 5 ppm, a rhodamine (III) content of less than 5 ppm and a rhodamine hexyl ester (IV) content of less than 5 ppm; even more preferred a PSDHE (II) content of less than 5 ppm, a rhodamine (III) content of less than 1 ppm and a rhodamine hexyl ester (IV) content of less than 1 ppm and most preferred a PSDHE (II) content of less than 1 ppm, a rhodamine (III) content of less than 1 ppm and a rhodamine hexyl ester (IV) content of less than 1 ppm; each based on the weight of the purified diethylamino hydroxybenzoyl hexyl benzoate (I).

Certain other combinations of by-products in the purified diethylamino hydroxybenzoyl hexyl benzoate (I) obtained by the inventive methods are also particularly preferable. In particular, it is preferred that the inventive purified diethylamino hydroxybenzoyl hexyl benzoate (I) has a PSDHE (II) content of less than 120 ppm, a rhodamine (III) content of less than 10 ppm, a rhodamine hexyl ester (IV) content of less than 10 ppm, and a 2-(4'-diethylamino-2'-hydroxybenzoyl)benzoic acid (V) content of less than 100 ppm; more preferred a PSDHE (II) content of less than 5 ppm, a rhodamine (III) content of less than 5 ppm, a rhodamine hexyl ester (IV) content of less than 5 ppm and a 2-(4'-diethylamino-2'-hydroxybenzoyl)benzoic acid (V) content of less than 60 ppm; even more preferred a PSDHE (II) content of less than 5 ppm, a rhodamine (III) content of less than 1 ppm, a rhodamine hexyl ester (IV) content of less than 1 ppm and a 2-(4'-diethylamino-2'-hydroxybenzoyl)benzoic acid (V) content of less than 10 ppm; and most preferred a PSDHE (II) content of less than 1 ppm, a rhodamine (III) content of less than 1 ppm, a rhodamine hexyl ester (IV) content of less than 1 ppm and a 2-(4'-diethylamino-2'-hydroxybenzoyl)benzoic acid (V) content of less than 1 ppm; each based on the weight of the crystalline diethylamino hydroxybenzoyl hexyl benzoate (I).

Another preferred embodiment of the purified diethylamino hydroxybenzoyl hexyl benzoate (I) obtained by the inventive methods has a PSDHE (II) content of less than 120 ppm, a rhodamine (III) content of less than 1 ppm, a rhodamine hexyl ester (IV) content of less than 1 ppm, and a 2-(4'-diethylamino-2'-hydroxybenzoyl)benzoic acid (V) content of less than 15 ppm, each based on the weight of the crystalline diethylamino hydroxybenzoyl hexyl benzoate (I).

Another aspect of the invention relates to a cosmetic composition comprising the inventive purified diethylamino hydroxybenzoyl hexyl benzoate (I). It is preferred that the purified DHHB in the cosmetic composition is in form of pourable/flowable particles, pastilles or flakes.

In a preferred embodiment, the cosmetic composition further comprises additives and adjuvants.

In a preferred embodiment, the cosmetic composition can be in the form of creams, gels, lotions, alcoholic and aqueous/alcoholic solutions, emulsions, wax/fat compositions, stick preparations, powders or ointments.

In a preferred embodiment, the cosmetic composition further comprises adjuvants and additives selected from mild surfactants, super-fatting agents, pearlescent waxes, consistency regulators, thickeners, polymers, silicone compounds, fats, waxes, stabilisers, biogenic active ingredients, deodorising active ingredients, anti-dandruff agents, film formers, swelling agents, antioxidants, hydrotropic agents, preservatives, insect repellents, self-tanning agents, solubilizers, perfume oils, colorants, bacteria-inhibiting agents and the like.

In a preferred embodiment, the cosmetic compositions according to the presently claimed invention may further comprise as adjuvants, anti-foams, structurants, solubilizers, opacifiers, complexing agents, propellants, coupler and developer components as oxidation dye precursors, reducing agents and oxidizing agents.

In a preferred embodiment, the cosmetic compositions are contained in a wide variety of cosmetic preparations, especially the following preparations:
- skin-care preparations, e.g. skin-washing and cleansing preparations in the form of tablet-form or liquid soaps, synthetic detergents or washing pastes,
- bath preparations, e.g. liquid (foam baths, milks, shower preparations) or solid bath preparations, e.g. bath cubes and bath salts;
- skin-care preparations, e.g. skin emulsions, multi-emulsions or skin oils;
- cosmetic personal care preparations, e.g. facial make-up in the form of day creams or powder creams, face powder (loose or pressed), rouge or cream make-up, eye-care preparations, e.g. eye shadow preparations, mascara, eyeliner, eye creams or eye-fix creams; lip-care preparations, e.g. lipsticks, lip gloss, lip contour pencils, nail-care preparations, such as nail varnish, nail varnish removers, nail hardeners or cuticle removers;
- foot-care preparations, e.g. foot baths, foot powders, foot creams or foot balsams, special deodorants and antiperspirants or callus-removing preparations;
- light-protective preparations, such as sun milks, lotions, creams or oils, sunblocks or tropicals, pre-tanning preparations or after-sun preparations;
- skin-tanning preparations, e.g. self-tanning creams;
- depigmenting preparations, e.g. preparations for bleaching the skin or skin-lightening preparations;
- insect-repellents, e.g. insect-repellent oils, lotions, sprays or sticks;
- deodorants, such as deodorant sprays, pump-action sprays, deodorant gels, sticks or roll-ons;
- antiperspirants, e.g. antiperspirant sticks, creams or roll-ons;
- preparations for cleansing and caring for blemished skin, e.g. synthetic detergents (solid or liquid), peeling or scrub preparations or peeling masks;
- hair-removal preparations in chemical form (depilation), e.g. hair-removing powders, liquid hair-removing preparations, cream- or paste-form hair-removing preparations, hair-removing preparations in gel form or aerosol foams;
- shaving preparations, e.g. shaving soap, foaming shaving creams, non-foaming shaving creams, foams and gels, preshave preparations for dry shaving, aftershaves or aftershave lotions;
- fragrance preparations, e.g. fragrances (eau de Cologne, eau de toilette, eau de parfum, parfum de toilette, parfume), parfume oils or parfume creams;
- cosmetic hair-treatment preparations, e.g. hair-washing preparations in the form of shampoos and conditioners, hair-care preparations, e.g. pre-treatment preparations, hair tonics, styling creams, styling gels, pomades, hair rinses, treatment packs, intensive hair treatments, hair-structuring preparations, e.g. hair-waving preparations for permanent waves (hot wave, mild wave, cold wave), hair-straightening preparations, liquid hair-setting preparations, hair foams, hairsprays, bleaching preparations, e.g. hydrogen peroxide solutions, lightening shampoos, bleaching creams, bleaching powders, bleaching pastes or oils, temporary, semi-permanent or permanent hair colorants, preparations containing self-oxidising dyes, or natural hair colorants, such as henna or camomile.

All statements made regarding the inventive purified DHHB herein also apply to the purified DHHB comprised within the inventive cosmetic composition.

The presently claimed invention offers one or more of the following advantages:
1) The method of the presently claimed invention provides crystalline DHHB having a high purity and a low amount of impurities (II), (III), (IV), and (V). The method of the presently claimed invention provides crystalline DHHB having less than 10 ppm (II). A low amount of (II) is relevant in view of regulatory requirements.
2) The method of the presently claimed invention is fast.
3) The method of the presently claimed invention is efficient as it provides a high yield of DHHB; in the range of 85 to 99 % based on the total of DHHB in the feed.
4) The crystalline DHHB obtained by the process of the presently claimed invention has a low amount of fines.

### Examples

### General

2-(4'-Diethylamino-2'-hydroxybenzoyl) benzoic acid hexyl ester (I) in hexanol was prepared according to US20050165099, example 2.

The product obtained from the process was subjected to crystallization as described in the experiments.

### Methods

HPLC method for determination of 2-(4'-diethylamino-2'-hydroxybenzoyl) benzoic acid hexyl ester (I), phthalate esters (II), and rhodamine type compounds (III) and (IV) as well as of 2-(4'-diethylamino-2'-hydroxybenzoyl)benzoic acid have been performed by standard state of the art methods.

GC method for determination of phthalate esters and solvent residues have been performed by standard state of the art methods.

Analytical standards for calibration have been performed by standard state of the art methods.

### Comparative examples according to WO 03097578

### Comparative Example C1: Crystallization according to WO 03097578

664.3 g of feed comprising 49.0 wt% 2-(4'-diethylamino-2'-hydroxybenzoyl)benzoic acid hexyl ester in hexanol containing 0.74 wt% di-n-hexylphthalate, 0.14 wt% rhodamine and 0.32 wt% rhodamine-hexyl ester corresponding to 14847 ppm di-n-hexylpthalate, 2786 ppm rhodamine and 6397 ppm rhodamine-hexylester calculated on 2-(4'-diethylamino-2'-hydroxybenzoyl)benzoic acid hexyl ester (s. above) was charged to a 1.6 L reactor.

The feed was heated at 40°C to obtain a solution. The solution was cooled to 20°C within 1.3 h to obtain a supersaturated solution having a c:c* ratio of 5.4.

After 4 h at 20°C crystallization started. The suspension was stirred at 20°C overnight (12 h), further cooled to 0°C within 4 h and stirred at 0°C. The product was filtered off and washed with a portion of 183 g pre-cooled (0°C) 1-hexanol and followed by another portion of 200 g pre-cooled (0°C) 1-hexanol to provide 371.5 g hexanol-wet 2-(4'-diethylamino-2'-hydroxybenzoyl)benzoic acid hexyl ester with a content of 80.6 wt% 2-(4'-diethylamino-2'-hydroxybenzoyl)benzoic acid hexyl ester.

Byproducts content calculated on the basis of (I): 110 ppm rhodamine (III), 131 ppm rhodamine-hexylester (IV) and 271 ppm di-n-hexylphthalate (II).

### Comparative Example C2: Crystallization according to WO 03097578

605.6 g of feed comprising 45.0 wt% 2-(4'-diethylamino-2'-hydroxybenzoyl)benzoic acid hexyl ester in hexanol containing 0.71 wt% di-n-hexylphthalate, 0.06 wt% rhodamine and 0.33 wt% rhodamine-hexylester corresponding to 15546 ppm di-n-hexylpthalate, 1298 ppm rhodamine and 7292 ppm rhodamine-hexylester calculated on 2-(4'-diethylamino-2'-hydroxybenzoyl)benzoic acid hexyl ester were charged to a 1.6 I reactor and the feed was heated at 40°C to obtain a solution.

The solution was cooled to 20°C within 1 h to obtain a supersaturated solution having a c:c* ratio of c:c* 5.0.

Crystallization started after 3.5 h at 20°C. The mixture was stirred for one more hour at 20°C followed by cooling to 0°C within 4 h and stirred at 0°C. The product was filtered off and washed with a first portion of 169 g pre-cooled (0°C) 1-hexanol followed by a second portion of 183 g pre-cooled (0°C) 1-hexanol to provide 310.9 g hexanol-wet 2-(4'-diethylamino-2'-hydroxybenzoyl)benzoic acid hexyl ester with a content of 79.1 wt% 2-(4'-diethylamino-2'-hydroxybenzoyl)benzoic acid hexyl ester.

Byproducts content calculated on the basis of (I): 73 ppm rhodamine (III), 187 ppm rhodamine-hexylester (IV) and 344 ppm di-n-hexylphthalate (II).

### Example 1: Crystallization

659 g of feed comprising 45.9 wt% 2-(4'-diethylamino-2'-hydroxybenzoyl)benzoic acid hexyl ester in hexanol containing 2.28 wt% di-n-hexylphthalate, 0.07 wt% rhodamine, 0.26 wt% rhodamine-hexylester and 0.01 wt% 2-(4'-diethylamino-2'-hydroxybenzoyl)benzoic acid corresponding to 47385 ppm di-n-hexylpthalate, 1555 ppm rhodamine, 5724 ppm rhodamine-hexylester and 218 ppm 2-(4'-diethylamino-2'-hydroxybenzoyl)benzoic acid calculated on 2-(4'-diethylamino-2'-hydroxybenzoyl)benzoic acid hexyl ester was charged to a 1.6 L reactor and the feed was heated at 60°C to obtain a solution.

The solution was cooled to 25°C within 1.0 h to obtain a supersaturated solution having c:c* ratio of 3.2.

The supersaturated solution was seeded with 5.2 g (1.5 wt% cal. on I) 2-(4'-diethylamino-2'-hydroxybenzoyl)benzoic acid hexyl ester (87.2 wt%) followed by stirring for 3.5 h at 25°C. The suspension was further cooled to 0°C within 5 h and stirred at 0°C. The product was filtered off and washed with a first portion of 187.4 g pre-cooled 1-hexanol (0°C) followed by a second portion of 202.5 g pre-cooled 1-hexanol (0°C, 389,9 g in total) to provide 309.8 g hexanol-wet 2-(4'-diethylamino-2'-hydroxybenzoyl)benzoic acid hexyl ester (I) with a content of 85.3 wt% 2-(4'-diethylamino-2'-hydroxybenzoyl)benzoic acid hexyl ester (I).

Byproducts content calculated on basis of (I): 246 ppm di-n-hexylphthalate (II), 35 ppm rhodamine (III), 68 ppm rhodamine-hexylester (IV) and 5 ppm 2-(4'-diethylamino-2'-hydroxybenzoyl)benzoic acid (V).

### Example 2: Recrystallization of crystalline (I) obtained in example 1

278.4 g of the hexanol wet filter cake obtained in example 1 comprising 2-(4'-diethylamino-2'-hydroxybenzoyl)benzoic acid hexyl ester (85.3 wt%) and 235 g hexanol were charged to a 1.6 L reactor and the feed was heated to 60°C to obtain a solution.

The solution was cooled to 25°C within 1.0 h to obtain a supersaturated solution having c:c* ratio of 2.9.

The supersaturated solution was seeded with 4.1 g (1.5 wt% cal. on I) 2-(4'-diethylamino-2'-hydroxybenzoyl)benzoic acid hexyl ester (87.2 wt%) followed by stirring for 3.5 h at 25°C. The suspension was further cooled to 0°C within 5 h and stirred at 0°C. The suspension was filtered and the product was washed three times with 185.2 g pre-cooled 1-hexanol (0°C, 555,6 g in total) to provide 232.3 g hexanol-wet 2-(4'-diethylamino-2'-hydroxybenzoyl)benzoic acid hexyl ester with a content of 82.9 wt% 2-(4'-diethylamino-2'-hydroxybenzoyl)benzoic acid hexyl ester.

Byproducts content cal. on (I): 0,4 ppm di-n-hexylphthalate (II), 3 ppm rhodamine (III), 2 ppm rhodamine-hexylester (IV) and 1 ppm 2-(4'-diethylamino-2'-hydroxybenzoyl)benzoic acid (V).

### Example 3: Crystallization

687 g of feed comprising 44.0 wt% 2-(4'-diethylamino-2'-hydroxybenzoyl)benzoic acid hexyl ester in hexanol containing 0.63 wt% di-n-hexylphthalate, 0.10 wt% rhodamine, 0.29 wt% rhodamine-hexylester and <0.01 wt% 2-(4'-diethylamino-2'-hydroxybenzoyl)benzoic acid corresponding to 14187 ppm di-n-hexylpthalate, 2294 ppm rhodamine, 6501 ppm rhodamine-hexylester and 12 ppm 2-(4'-diethylamino-2'-hydroxybenzoyl)benzoic acid calculated on 2-(4'-diethylamino-2'-hydroxybenzoyl)benzoic acid hexyl ester was charged to a 1.6 L reactor and the feed was heated to 60°C to obtain a solution.

The solution was cooled to 25°C within 1.0 h to obtain a supersaturated solution having c:c* ratio of 3.0.

The supersaturated solution was seeded with 5.2 g (1.5 wt% cal. on I) 2-(4'-diethylamino-2'-hydroxybenzoyl)benzoic acid hexyl ester (87.2 wt%) followed by stirring for 3.5 h at 25°C. The suspension was further cooled to 0°C within 5 h and stirred at 0°C. The product was filtered off and washed four times with 241.8 g pre-cooled 1-hexanol (0°C, 967.2 g 1-hexanol in total) to provide 266.4 g hexanol-wet 2-(4'-diethylamino-2'-hydroxybenzoyl)benzoic acid hexyl ester (I) with a content of 84.9 wt% 2-(4'-diethylamino-2'-hydroxybenzoyl)benzoic acid hexyl ester (I).

Byproducts content calculated on basis of (I): 36 ppm di-n-hexylphthalate (II), 34 ppm rhodamine (III), 61 ppm rhodamine-hexylester (IV) and 3 ppm 2-(4'-diethylamino-2'-hydroxybenzoyl)benzoic acid (V).

### Example 4: Recrystallization of crystalline (I) obtained in example 3

237.4 g of the hexanol wet filter cake obtained in example 3 comprising 2-(4'-diethylamino-2'-hydroxybenzoyl)benzoic acid hexyl ester (84.9 wt%) and 200 g hexanol were charged to a 1.6 L reactor and the feed was heated at 60°C to obtain a solution.

The solution was cooled to 25°C within 1.0 h to obtain a supersaturated solution having c:c* ratio of 2.9. dito

The supersaturated solution was seeded with 3.4 g (1.5 wt% cal. on I) 2-(4'-diethylamino-2'-hydroxybenzoyl)benzoic acid hexyl ester (87.2 wt%) followed by stirring for 3.5 h at 25°C. The suspension was further cooled to 0°C within 5 h and stirred at 0°C. The suspension was filtered and the product washed with a first portion of 125.0 g pre-cooled (0°C) 1-hexanol and a second time by 135.1 g pre-cooled 1-hexanol (0°C, 260.1 g in total) to provide 209.5 g hexanol-wet 2-(4'-diethylamino-2'-hydroxybenzoyl)benzoic acid hexyl ester with a content of 84.3 wt% 2-(4'-diethylamino-2'-hydroxybenzoyl)benzoic acid hexyl ester.

Byproducts content cal. on (I): <1 ppm di-n-hexylphthalate (II), 3 ppm rhodamine (III), 2 ppm rhodamine-hexylester (IV) and 1 ppm 2-(4'-diethylamino-2'-hydroxybenzoyl)benzoic acid (V).

### Conclusion

As demonstrated by the Examples, the inventive method allows for the preparation of clean crystalline DHHB (I) with very low amounts of by-products compared to previous methods of crystallizing DHHB.

## Claims

1. A method for obtaining crystalline diethylamino hydroxybenzoyl hexyl benzoate (I) comprising steps (a) to (d),
(a) providing a feed comprising diethylamino hydroxybenzoyl hexyl benzoate (I) and at least a first crystallization solvent, and heating the feed to obtain a solution, wherein the diethylamino hydroxybenzoyl hexyl benzoate (I) has
i) a phthalic acid dihexyl ester (PSDHE) (II) content of up to 100000 ppm;
based on the weight of diethylamino hydroxybenzoyl hexyl benzoate (I); and
wherein the amount of the diethylamino hydroxybenzoyl hexyl benzoate (I) in the feed is in the range of 15.0 to 65.0 wt% based on the total weight of the feed;
(b) cooling the solution obtained in step (a) to obtain a supersaturated solution of diethylamino hydroxybenzoyl hexyl benzoate (I);
(c) seeding the supersaturated solution obtained in step (b) with seed crystals of diethylamino hydroxybenzoyl hexyl benzoate (I);
wherein the amount of the seed crystals is in the range from 0.01 to 15.0 wt% based on the weight of diethylamino hydroxybenzoyl hexyl benzoate (I) in the solution; and
(d) cooling the solution obtained in step (c) to a temperature in the range of -20 to 17 ° C to obtain a suspension comprising crystalline diethylamino hydroxybenzoyl hexyl benzoate (I); followed by separating crystalline diethylamino hydroxybenzoyl hexyl benzoate (I) and washing the separated crystalline diethylamino hydroxybenzoyl hexyl benzoate (I) with a washing solvent at least a first time and a second time;
and
(e) optionally recrystallizing the diethylamino hydroxybenzoyl hexyl benzoate (I) obtained in step (d) involving mixing the diethylamino hydroxybenzoyl hexyl benzoate (I) obtained in step (d) with at least a second crystallization solvent to obtain a feed, heating the feed to obtain a solution, and using the method steps (b) to (d);
wherein the quantity of washing solvent is in each of the at least first time and second time washing in at least one of step (d) of the crystallization or step (d) of the recrystallization (e) at least 63 wt.%, based on the weight of the diethylamino hydroxybenzoyl hexyl benzoate (I) in the corresponding solution in step (b), wherein the first crystallization solvent comprises at least one C₄-C₈ alcohol.

2. A method for obtaining crystalline diethylamino hydroxybenzoyl hexyl benzoate (I) comprising steps (a) to (d),
(a) providing a feed comprising diethylamino hydroxybenzoyl hexyl benzoate (I) and at least a first crystallization solvent, and heating the feed to obtain a solution, wherein the diethylamino hydroxybenzoyl hexyl benzoate (I) has
i) a phthalic acid dihexyl ester (PSDHE) (II) content of up to 100000 ppm;
based on the weight of diethylamino hydroxybenzoyl hexyl benzoate (I); and
wherein the amount of the diethylamino hydroxybenzoyl hexyl benzoate (I) in the feed is in the range of 15.0 to 65.0 wt% based on the total weight of the feed;
(b) cooling the solution obtained in step (a) to obtain a supersaturated solution of diethylamino hydroxybenzoyl hexyl benzoate (I);
(c) seeding the supersaturated solution obtained in step (b) with seed crystals of diethylamino hydroxybenzoyl hexyl benzoate (I);
wherein the amount of the seed crystals is in the range from 0.01 to 15.0 wt% based on the weight of diethylamino hydroxybenzoyl hexyl benzoate (I) in the solution; and
(d) cooling the solution obtained in step (c) to a temperature in the range of -20 to 17 ° C to obtain a suspension comprising crystalline diethylamino hydroxybenzoyl hexyl benzoate (I); followed by separating crystalline diethylamino hydroxybenzoyl hexyl benzoate (I) and washing the separated crystalline diethylamino hydroxybenzoyl hexyl benzoate (I) with a washing solvent;
and
(e) optionally recrystallizing the diethylamino hydroxybenzoyl hexyl benzoate (I) obtained in step (d) involving mixing the diethylamino hydroxybenzoyl hexyl benzoate (I) obtained in step (d) with the at least a second crystallization solvent to obtain a feed, heating the feed to obtain a solution, and using the method steps (b) to (d);
wherein the quantity of washing solvent is in at least one of step (d) of the crystallization or step (d) of the recrystallization (e) at least 130 wt.%, based on the weight of the diethylamino hydroxybenzoyl hexyl benzoate (I) in the corresponding solution in step (b)
wherein the first crystallization solvent comprises at least one C₄-C₈ alcohol.

3. The method according to one of claims 1 or 2 **characterized in that** the solution in step (b) is cooled to a temperature such that the ratio of c : c* of the concentration c of dissolved diethylamino hydroxybenzoyl hexyl benzoate (I) to the equilibrium solubility c* of diethylamino hydroxybenzoyl hexyl benzoate (I) at the temperature is in the range from 1.1:1.0 to 9.0:1.0 and/or **in that** the diethylamino hydroxybenzoyl hexyl benzoate (I) in step (a) has a phthalic acid dihexyl ester (PSDHE) (II) content of 10 to 70000 ppm, in particular from 500 to 50000 ppm or from 5000 to 40000 or from 7000 to 30000 ppm, based on the weight of diethylamino hydroxybenzoyl hexyl benzoate (I) and/or **in that** after seeding the supersaturated solution obtained in step (b) with seed crystals of diethylamino hydroxybenzoyl hexyl benzoate (I), the supersaturated solution comprising seed crystals is agitated for up to 8 hours, in particular 20 minutes to 7 hours, before cooling in step (d).

4. The method according to any one of the preceding claims **characterized in that** the first and/or second crystallization solvent comprises or consists of at least one selected from the group consisting of C₄-C₈ alcohols and/or **in that** the first and/or second crystallization solvent comprises or consists of 1-hexanol.

5. The method according to any one of the preceding claims **characterized in that** the washing solvent comprises an alcohol, in particular selected from the group consisting of C₁-C₈ alcohols, in particular methanol, ethanol, iso-propanol, n-propanol, iso-butanol, tert-butanol, n-butanol, n-pentanol, sec-pentanol, cyclopentanol, n-hexanol, sec-hexanol, cyclohexanol, n-heptanol, sec-heptanol, n-octanol, sec-octanol.

6. The method according to any one of the preceding claims **characterized in that** the washing solvent is different from the first and/or the second crystallization solvent or **in that** the washing solvent and the first and/or the second crystallization solvent are the same.

7. The method according to any one of the preceding claims **characterized in that** the washing is performed by displacement wash and/or by reslurry/suspension wash and/or **in that** in step (a) the feed is heated to a temperature in the range of 25 to 80 ° C, in particular to the range of 35 to 60 ° C and/or **in that** in step (b) the solution is cooled to a temperature in the range of by 20 to 35 ° C and/or **in that** in step (c) the seeding is carried out at a temperature in the range of 20 to 35 ° C and/or **in that** in step (d) the solution is cooled at a cooling rate of 1 to 15 K/h.

8. The method according to any one of the preceding claims **characterized in that** in step (d) the solution is cooled in a linear manner or **in that** in step (d) the solution is cooled with a piecewise linear cooling profile or **in that** in step (d) the solution is cooled with a parabolic cooling profile with increasing cooling rates or **in that** in step (d) the solution is cooled with a dynamic cooling profile to maintain a relatively constant supersaturation in the crystallizer or **in that** in step (d) the solution is cooled with vacuum cooling method.

9. The method according to any one of the preceding claims **characterized in that** in step (d) the separation of crystals is carried out by filtration while maintaining the temperature of the solution within ± 5 ° C of the temperature to which the solution is cooled in step (d) and/or **in that** the washing of the separated crystalline diethylamino hydroxybenzoyl hexyl benzoate (I) is carried with washing solvent having a temperature within ± 5 ° C of the temperature to which the solution is cooled in step (d) and/or **in that** the crystallization is carried out in a crystallizer selected from a stirred vessel with baffles, force circulation crystallizer, draft tube crystallizer, draft tube baffled crystallizer, and an Oslo type crystallizer.

10. The method according to any one of the preceding claims **characterized in that** the method further comprises the following steps:
i. dissolving the crystalline diethylamino hydroxybenzoyl hexyl benzoate (I) obtained in step (d) or step (e) in a purification solvent;
ii. contacting the solution obtained in step (i) with an adsorbent; and
iii. removal of solvent from the solution obtained in step (ii) to obtain purified diethylamino hydroxybenzoyl hexyl benzoate (I).

11. The method according to claim 10 **characterized in that** the purification solvent is selected from toluene, cyclohexane and combinations thereof and/or the adsorbent is selected from silica, charcoal and combinations thereof.

12. Crystalline diethylamino hydroxybenzoyl hexyl benzoate (I) obtained by the method according to any of claims 1 to 9 having
i) a phthalic acid dihexyl ester (PSDHE) 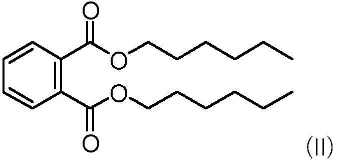 content of less than 250 ppm;
ii) a rhodamine 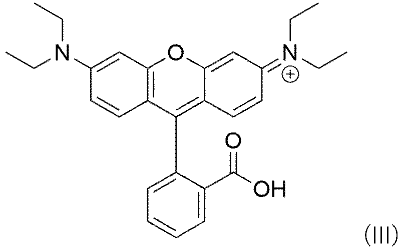 content of less than 150 ppm; and
iii) a rhodamine hexyl ester 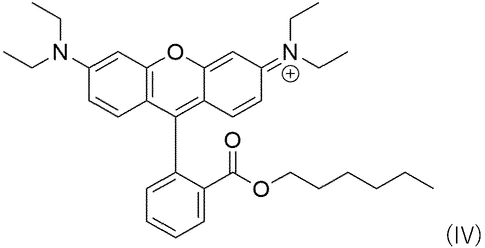 content of less than 150 ppm;
each based on the weight of the crystalline diethylamino hydroxybenzoyl hexyl benzoate (I).

13. The crystalline diethylamino hydroxybenzoyl hexyl benzoate (I) of claim 12 **characterized in that** the phthalic acid dihexyl ester (PSDHE) 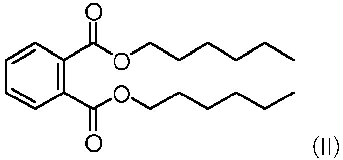 content is less than 1 ppm and/or the rhodamine 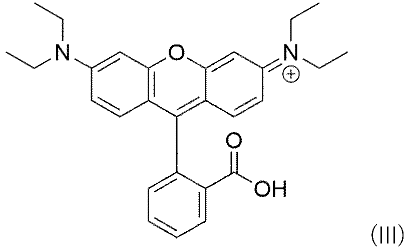 content is less than 10 ppm and/or the rhodamine hexyl ester 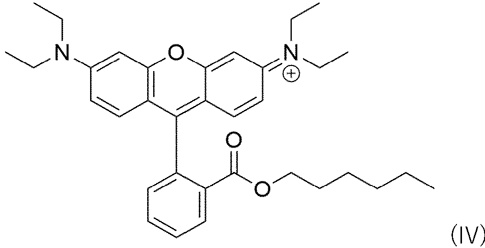 content is less than 10 ppm, each based on the weight of the crystalline diethylamino hydroxybenzoyl hexyl benzoate (I).

14. Purified diethylamino hydroxybenzoyl hexyl benzoate (I) obtained by the method according to claim 10 or 11 having
i) a phthalic acid dihexyl ester (PSDHE) 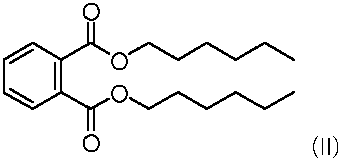 content of less than 250 ppm;
ii) a rhodamine 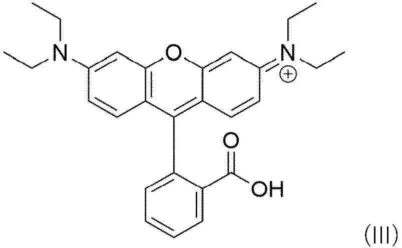 content of less than 10 ppm; and
iii) a rhodamine hexyl ester 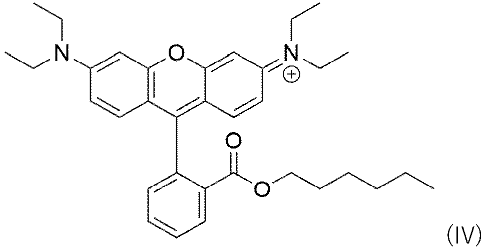 content of less than 10 ppm;
each based on the weight of the purified diethylamino hydroxybenzoyl hexyl benzoate (I).

15. A cosmetic composition comprising the purified diethylamino hydroxybenzoyl hexyl benzoate (I) of claim 14.

## Patentansprüche

1. Verfahren zur Gewinnung von kristallinem Diethylaminohydroxybenzoylhexylbenzoat (I), umfassend die Schritte (a) bis (d),
(a) Bereitstellen eines Einsatzgemisches, umfassend Diethylaminohydroxybenzoylhexylbenzoat (I) und mindestens ein erstes Kristallisationslösungsmittel, und Erwärmen des Einsatzgemisches, um eine Lösung zu erhalten, wobei das Diethylaminohydroxybenzoylhexylbenzoat (I)
i) einen Gehalt an Phthalsäuredihexylester (PSDHE) (II) von bis zu 100000 ppm, bezogen auf das Gewicht von Diethylaminohydroxybenzoylhexylbenzoat (I), aufweist; und
wobei die Menge des Diethylaminohydroxybenzoylhexylbenzoats (I) in dem Einsatzgemisch im Bereich von 15,0 bis 65,0 Gew.-%, bezogen auf das Gesamtgewicht des Einsatzgemisches, liegt;
(b) Abkühlen der in Schritt (a) erhaltenen Lösung, um eine übersättigte Lösung von Diethylaminohydroxybenzoylhexylbenzoat (I) zu erhalten;
(c) Animpfen der in Schritt (b) erhaltenen übersättigten Lösung mit Impfkristallen von Diethylaminohydroxybenzoylhexylbenzoat (I);
wobei die Menge der Impfkristalle im Bereich von 0,01 bis 15,0 Gew.-%, bezogen auf das Gewicht von Diethylaminohydroxybenzoylhexylbenzoat (I) in der Lösung, liegt; und
(d) Abkühlen der in Schritt (c) erhaltenen Lösung auf eine Temperatur im Bereich von -20 bis 17 °C, um eine Suspension zu erhalten, die kristallines Diethylaminohydroxybenzoylhexylbenzoat (I) umfasst; gefolgt von Abtrennen von kristallinem Diethylaminohydroxybenzoylhexylbenzoat (I) und Waschen des abgetrennten kristallinen Diethylaminohydroxybenzoylhexylbenzoats (I) mit einem Waschlösungsmittel mindestens ein erstes Mal und ein zweites Mal;
und
(e) optional Umkristallisieren des in Schritt (d) erhaltenen Diethylaminohydroxybenzoylhexylbenzoats (I), umfassend Mischen des in Schritt (d) erhaltenen Diethylaminohydroxybenzoylhexylbenzoats (I) mit mindestens einem zweiten Kristallisationslösungsmittel, um ein Einsatzgemisch zu erhalten, Erwärmen des Einsatzgemisches, um eine Lösung zu erhalten, und Anwenden der Verfahrensschritte (b) bis (d); wobei die Menge an Waschlösungsmittel bei jeder der mindestens ersten und zweiten Waschung in mindestens einem von Schritt (d) der Kristallisation oder Schritt (d) der Umkristallisation (e) mindestens 63 Gew.-%, bezogen auf das Gewicht des Diethylaminohydroxybenzoylhexylbenzoats (I) in der entsprechenden Lösung in Schritt (b), beträgt,
wobei das erste Kristallisationslösungsmittel mindestens einen C₄-C₈-Alkohol umfasst.

2. Verfahren zur Gewinnung von kristallinem Diethylaminohydroxybenzoylhexylbenzoat (I), umfassend die Schritte (a) bis (d),
(a) Bereitstellen eines Einsatzgemisches, umfassend Diethylaminohydroxybenzoylhexylbenzoat (I) und mindestens ein erstes Kristallisationslösungsmittel, und Erwärmen des Einsatzgemisches, um eine Lösung zu erhalten,
wobei das Diethylaminohydroxybenzoylhexylbenzoat (I)
i) einen Gehalt an Phthalsäuredihexylester (PSDHE) (II) von bis zu 100000 ppm, bezogen auf das Gewicht von Diethylaminohydroxybenzoylhexylbenzoat (I), aufweist; und
wobei die Menge des Diethylaminohydroxybenzoylhexylbenzoats (I) in dem Einsatzgemisch im Bereich von 15,0 bis 65,0 Gew.-%, bezogen auf das Gesamtgewicht des Einsatzgemisches, liegt;
(b) Abkühlen der in Schritt (a) erhaltenen Lösung, um eine übersättigte Lösung von Diethylaminohydroxybenzoylhexylbenzoat (I) zu erhalten;
(c) Animpfen der in Schritt (b) erhaltenen übersättigten Lösung mit Impfkristallen von Diethylaminohydroxybenzoylhexylbenzoat (I);
wobei die Menge der Impfkristalle im Bereich von 0,01 bis 15,0 Gew.-%, bezogen auf das Gewicht von Diethylaminohydroxybenzoylhexylbenzoat (I) in der Lösung, liegt; und
(d) Abkühlen der in Schritt (c) erhaltenen Lösung auf eine Temperatur im Bereich von -20 bis 17 °C, um eine Suspension zu erhalten, die kristallines Diethylaminohydroxybenzoylhexylbenzoat (I) umfasst; gefolgt von Abtrennen von kristallinem Diethylaminohydroxybenzoylhexylbenzoat (I) und Waschen des abgetrennten kristallinen Diethylaminohydroxybenzoylhexylbenzoats (I) mit einem Waschlösungsmittel;
und
(e) optional Umkristallisieren des in Schritt (d) erhaltenen Diethylaminohydroxybenzoylhexylbenzoats (I), umfassend Mischen des in Schritt (d) erhaltenen Diethylaminohydroxybenzoylhexylbenzoats (I) mit dem mindestens einen zweiten Kristallisationslösungsmittel, um ein Einsatzgemisch zu erhalten, Erwärmen des Einsatzgemisches, um eine Lösung zu erhalten, und Anwenden der Verfahrensschritte (b) bis (d);
wobei die Menge an Waschlösungsmittel in mindestens einem von Schritt (d) der Kristallisation oder Schritt (d) der Umkristallisation (e) mindestens 130 Gew.-%, bezogen auf das Gewicht des Diethylaminohydroxybenzoylhexylbenzoats (I) in der entsprechenden Lösung in Schritt (b), beträgt, wobei das erste Kristallisationslösungsmittel mindestens einen C₄-C₈-Alkohol umfasst.

3. Verfahren nach einem der Ansprüche 1 oder 2, **dadurch gekennzeichnet, dass** die Lösung in Schritt (b) auf eine Temperatur abgekühlt wird, so dass das Verhältnis c : c* der Konzentration c von gelöstem Diethylaminohydroxybenzoylhexylbenzoat (I) zur Gleichgewichtslöslichkeit c* von Diethylaminohydroxybenzoylhexylbenzoat (I) bei der Temperatur im Bereich von 1,1:1,0 bis 9,0:1,0 liegt, und/oder dadurch, dass das Diethylaminohydroxybenzoylhexylbenzoat (I) in Schritt (a) einen Gehalt an Phthalsäuredihexylester (PSDHE) (II) von 10 bis 70000 ppm, insbesondere von 500 bis 50000 ppm oder von 5000 bis 40000 oder von 7000 bis 30000 ppm, bezogen auf das Gewicht von Diethylaminohydroxybenzoylhexylbenzoat (I), aufweist, und/oder dadurch, dass nach dem Animpfen der in Schritt (b) erhaltenen übersättigten Lösung mit Impfkristallen von Diethylaminohydroxybenzoylhexylbenzoat (I) die Impfkristalle umfassende übersättigte Lösung vor dem Abkühlen in Schritt (d) bis zu 8 Stunden, insbesondere 20 Minuten bis 7 Stunden, bewegt wird.

4. Verfahren nach einem der vorhergehenden Ansprüche, **dadurch gekennzeichnet, dass** das erste und/oder zweite Kristallisationslösungsmittel mindestens eines umfasst oder daraus besteht, ausgewählt aus der Gruppe bestehend aus C₄-C₈-Alkoholen, und/oder dadurch, dass das erste und/oder zweite Kristallisationslösungsmittel 1-Hexanol umfasst oder daraus besteht.

5. Verfahren nach einem der vorhergehenden Ansprüche, **dadurch gekennzeichnet, dass** das Waschlösungsmittel einen Alkohol umfasst, insbesondere ausgewählt aus der Gruppe bestehend aus C₁-C₈-Alkoholen, insbesondere Methanol, Ethanol, iso-Propanol, n-Propanol, iso-Butanol, tert-Butanol, n-Butanol, n-Pentanol, sec-Pentanol, Cyclopentanol, n-Hexanol, sec-Hexanol, Cyclohexanol, n-Heptanol, sec-Heptanol, n-Octanol, sec-Octanol.

6. Verfahren nach einem der vorhergehenden Ansprüche, **dadurch gekennzeichnet, dass** sich das Waschlösungsmittel von dem ersten und/oder dem zweiten Kristallisationslösungsmittel unterscheidet oder dadurch, dass das Waschlösungsmittel und das erste und/oder das zweite Kristallisationslösungsmittel gleich sind.

7. Verfahren nach einem der vorhergehenden Ansprüche, **dadurch gekennzeichnet, dass** das Waschen durch Verdrängungswäsche und/oder durch Reslurry-/Suspensionswäsche durchgeführt wird und/oder dadurch, dass in Schritt (a) das Einsatzgemisch auf eine Temperatur im Bereich von 25 bis 80 °C, insbesondere auf den Bereich von 35 bis 60 °C, erwärmt wird und/oder dadurch, dass in Schritt (b) die Lösung auf eine Temperatur im Bereich von 20 bis 35 °C abgekühlt wird und/oder dadurch, dass in Schritt (c) das Animpfen bei einer Temperatur im Bereich von 20 bis 35 °C durchgeführt wird und/oder dadurch, dass in Schritt (d) die Lösung mit einer Abkühlrate von 1 bis 15 K/h abgekühlt wird.

8. Verfahren nach einem der vorhergehenden Ansprüche, **dadurch gekennzeichnet, dass** in Schritt (d) die Lösung linear abgekühlt wird oder dass in Schritt (d) die Lösung mit einem abschnittsweise linearen Abkühlprofil abgekühlt wird oder dass in Schritt (d) die Lösung mit einem parabolischen Abkühlprofil mit zunehmenden Abkühlraten abgekühlt wird oder dass in Schritt (d) die Lösung mit einem dynamischen Abkühlprofil abgekühlt wird, um eine relativ konstante Übersättigung im Kristallisator aufrechtzuerhalten, oder dass in Schritt (d) die Lösung mittels Vakuumkühlverfahren abgekühlt wird.

9. Verfahren nach einem der vorhergehenden Ansprüche, **dadurch gekennzeichnet, dass** in Schritt (d) die Abtrennung der Kristalle durch Filtration durchgeführt wird, während die Temperatur der Lösung innerhalb von ± 5 °C der Temperatur gehalten wird, auf die die Lösung in Schritt (d) abgekühlt wird, und/oder dadurch, dass das Waschen des abgetrennten kristallinen Diethylaminohydroxybenzoylhexylbenzoats (I) mit Waschlösungsmittel durchgeführt wird, das eine Temperatur innerhalb von ± 5 °C der Temperatur aufweist, auf die die Lösung in Schritt (d) abgekühlt wird, und/oder dadurch, dass die Kristallisation in einem Kristallisator durchgeführt wird, ausgewählt aus einem gerührten Behälter mit Stromstörern, Zwangsumlaufkristallisator, Zugrohrkristallisator, Zugrohr-Stromstörer-Kristallisator und einem Kristallisator vom Oslo-Typ.

10. Verfahren nach einem der vorhergehenden Ansprüche, **dadurch gekennzeichnet, dass** das Verfahren ferner die folgenden Schritte umfasst:
i. Lösen des in Schritt (d) oder Schritt (e) erhaltenen kristallinen Diethylaminohydroxybenzoylhexylbenzoats (I) in einem Reinigungslösungsmittel;
ii. Inkontaktbringen der in Schritt (i) erhaltenen Lösung mit einem Adsorbens; und
iii. Entfernen von Lösungsmittel aus der in Schritt (ii) erhaltenen Lösung, um gereinigtes Diethylaminohydroxybenzoylhexylbenzoat (I) zu erhalten.

11. Verfahren nach Anspruch 10, **dadurch gekennzeichnet, dass** das Reinigungslösungsmittel ausgewählt ist aus Toluol, Cyclohexan und Kombinationen davon und/oder das Adsorbens ausgewählt ist aus Siliciumdioxid, Aktivkohle und Kombinationen davon.

12. Kristallines Diethylaminohydroxybenzoylhexylbenzoat (I), erhältlich durch das Verfahren nach einem der Ansprüche 1 bis 9, aufweisend
i) einen Gehalt an Phthalsäuredihexylester (PSDHE) 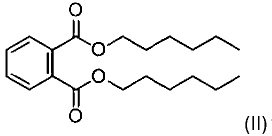 von weniger als 250 ppm;
ii) einen Gehalt an Rhodamin 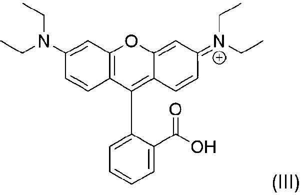 von weniger als 150 ppm; und
iii) einen Gehalt an Rhodaminhexylester 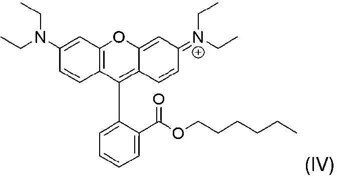 von weniger als 150 ppm; jeweils bezogen auf das Gewicht des kristallinen Diethylaminohydroxybenzoylhexylbenzoats (I).

13. Kristallines Diethylaminohydroxybenzoylhexylbenzoat (I) nach Anspruch 12, **dadurch gekennzeichnet,**
**dass** der Gehalt an Phthalsäuredihexylester (PSDHE) 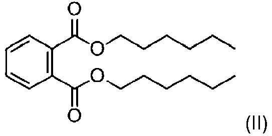 weniger als 1 ppm beträgt und/oder
der Gehalt an Rhodamin 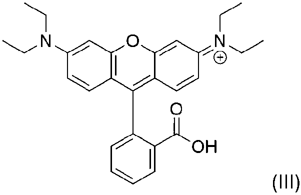 weniger als 10 ppm beträgt und/oder
der Gehalt an Rhodaminhexylester 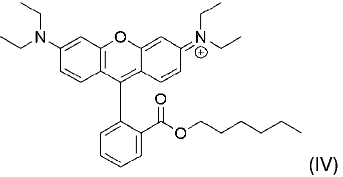 weniger als 10 ppm beträgt, jeweils bezogen auf das Gewicht des kristallinen Diethylaminohydroxybenzoylhexylbenzoats (I).

14. Gereinigtes Diethylaminohydroxybenzoylhexylbenzoat (I), erhältlich durch das Verfahren nach Anspruch 10 oder 11, aufweisend
i) einen Gehalt an Phthalsäuredihexylester (PSDHE) 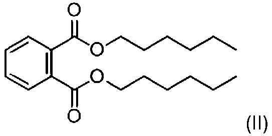 von weniger als 250 ppm;
ii) einen Gehalt an Rhodamin 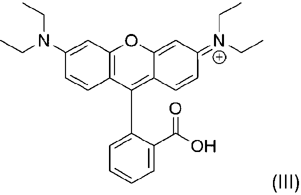 von weniger als 10 ppm; und
iii) einen Gehalt an Rhodaminhexylester 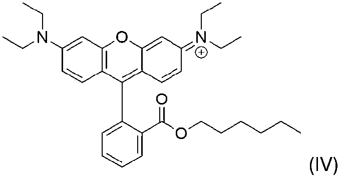 von weniger als 10 ppm; jeweils bezogen auf das Gewicht des gereinigten Diethylaminohydroxybenzoylhexylbenzoats (I).

15. Kosmetische Zusammensetzung, umfassend das gereinigte Diethylaminohydroxybenzoylhexylbenzoat (I) nach Anspruch 14.

## Revendications

1. Procédé d'obtention de diéthylamino hydroxybenzoyl hexyl benzoate (I) cristallin, comprenant les étapes (a) à (d),
(a) fournir une charge comprenant du diéthylamino hydroxybenzoyl hexyl benzoate (I) et au moins un premier solvant de cristallisation, et chauffer la charge afin d'obtenir une solution, dans lequel le diéthylamino hydroxybenzoyl hexyl benzoate (I) présente
i) une teneur en ester dihexylique de l'acide phtalique (PSDHE) (II) allant jusqu'à 100000 ppm, sur la base du poids de diéthylamino hydroxybenzoyl hexyl benzoate (I) ; et
dans lequel la quantité de diéthylamino hydroxybenzoyl hexyl benzoate (I) dans la charge est dans la plage de 15,0 à 65,0 % en poids sur la base du poids total de la charge ;
(b) refroidir la solution obtenue à l'étape (a) afin d'obtenir une solution sursaturée de diéthylamino hydroxybenzoyl hexyl benzoate (I) ;
(c) ensemencer la solution sursaturée obtenue à l'étape (b) avec des cristaux germes de diéthylamino hydroxybenzoyl hexyl benzoate (I) ;
dans lequel la quantité des cristaux germes est dans la plage de 0,01 à 15,0 % en poids sur la base du poids de diéthylamino hydroxybenzoyl hexyl benzoate (I) dans la solution ; et
(d) refroidir la solution obtenue à l'étape (c) à une température dans la plage de -20 à 17 °C afin d'obtenir une suspension comprenant du diéthylamino hydroxybenzoyl hexyl benzoate (I) cristallin ; suivi de la séparation du diéthylamino hydroxybenzoyl hexyl benzoate (I) cristallin et du lavage du diéthylamino hydroxybenzoyl hexyl benzoate (I) cristallin séparé avec un solvant de lavage au moins une première fois et une deuxième fois ;
et
(e) éventuellement recristalliser le diéthylamino hydroxybenzoyl hexyl benzoate (I) obtenu à l'étape (d), impliquant le mélange du diéthylamino hydroxybenzoyl hexyl benzoate (I) obtenu à l'étape (d) avec au moins un deuxième solvant de cristallisation afin d'obtenir une charge, le chauffage de la charge afin d'obtenir une solution, et l'utilisation des étapes de procédé (b) à (d) ;
dans lequel la quantité de solvant de lavage est, à chacune des au moins première et deuxième opérations de lavage dans au moins l'une de l'étape (d) de la cristallisation ou de l'étape (d) de la recristallisation (e), d'au moins 63 % en poids, sur la base du poids du diéthylamino hydroxybenzoyl hexyl benzoate (I) dans la solution correspondante à l'étape (b),
dans lequel le premier solvant de cristallisation comprend au moins un alcool en C4-C8.

2. Procédé d'obtention de diéthylamino hydroxybenzoyl hexyl benzoate (I) cristallin, comprenant les étapes (a) à (d),
(a) fournir une charge comprenant du diéthylamino hydroxybenzoyl hexyl benzoate (I) et au moins un premier solvant de cristallisation, et chauffer la charge afin d'obtenir une solution,
dans lequel le diéthylamino hydroxybenzoyl hexyl benzoate (I) présente
i) une teneur en ester dihexylique de l'acide phtalique (PSDHE) (II) allant jusqu'à 100000 ppm, sur la base du poids de diéthylamino hydroxybenzoyl hexyl benzoate (I) ; et
dans lequel la quantité de diéthylamino hydroxybenzoyl hexyl benzoate (I) dans la charge est dans la plage de 15,0 à 65,0 % en poids sur la base du poids total de la charge ;
(b) refroidir la solution obtenue à l'étape (a) afin d'obtenir une solution sursaturée de diéthylamino hydroxybenzoyl hexyl benzoate (I) ;
(c) ensemencer la solution sursaturée obtenue à l'étape (b) avec des cristaux germes de diéthylamino hydroxybenzoyl hexyl benzoate (I) ;
dans lequel la quantité des cristaux germes est dans la plage de 0,01 à 15,0 % en poids sur la base du poids de diéthylamino hydroxybenzoyl hexyl benzoate (I) dans la solution ; et
(d) refroidir la solution obtenue à l'étape (c) à une température dans la plage de -20 à 17 °C afin d'obtenir une suspension comprenant du diéthylamino hydroxybenzoyl hexyl benzoate (I) cristallin ; suivi de la séparation du diéthylamino hydroxybenzoyl hexyl benzoate (I) cristallin et du lavage du diéthylamino hydroxybenzoyl hexyl benzoate (I) cristallin séparé avec un solvant de lavage ;
et
(e) éventuellement recristalliser le diéthylamino hydroxybenzoyl hexyl benzoate (I) obtenu à l'étape (d), impliquant le mélange du diéthylamino hydroxybenzoyl hexyl benzoate (I) obtenu à l'étape (d) avec l'au moins un deuxième solvant de cristallisation afin d'obtenir une charge, le chauffage de la charge afin d'obtenir une solution, et l'utilisation des étapes de procédé (b) à (d) ;
dans lequel la quantité de solvant de lavage est, dans au moins l'une de l'étape (d) de la cristallisation ou de l'étape (d) de la recristallisation (e), d'au moins 130 % en poids, sur la base du poids du diéthylamino hydroxybenzoyl hexyl benzoate (I) dans la solution correspondante à l'étape (b),
dans lequel le premier solvant de cristallisation comprend au moins un alcool en C4-C8.

3. Procédé selon l'une des revendications 1 ou 2, **caractérisé en ce que** la solution à l'étape (b) est refroidie à une température telle que le rapport c : c* de la concentration c de diéthylamino hydroxybenzoyl hexyl benzoate (I) dissous à la solubilité d'équilibre c* du diéthylamino hydroxybenzoyl hexyl benzoate (I) à la température est dans la plage de 1,1:1,0 à 9,0:1,0 et/ou **en ce que** le diéthylamino hydroxybenzoyl hexyl benzoate (I) à l'étape (a) présente une teneur en ester dihexylique de l'acide phtalique (PSDHE) (II) de 10 à 70000 ppm, en particulier de 500 à 50000 ppm ou de 5000 à 40000 ou de 7000 à 30000 ppm, sur la base du poids de diéthylamino hydroxybenzoyl hexyl benzoate (I), et/ou **en ce qu'**après ensemencement de la solution sursaturée obtenue à l'étape (b) avec des cristaux germes de diéthylamino hydroxybenzoyl hexyl benzoate (I), la solution sursaturée comprenant des cristaux germes est agitée pendant jusqu'à 8 heures, en particulier de 20 minutes à 7 heures, avant le refroidissement à l'étape (d).

4. Procédé selon l'une quelconque des revendications précédentes, **caractérisé en ce que** le premier et/ou le deuxième solvant de cristallisation comprend ou consiste en au moins un composé choisi dans le groupe constitué par les alcools en C4-C8, et/ou **en ce que** le premier et/ou le deuxième solvant de cristallisation comprend ou consiste en 1-hexanol.

5. Procédé selon l'une quelconque des revendications précédentes, **caractérisé en ce que** le solvant de lavage comprend un alcool, en particulier choisi dans le groupe constitué par les alcools en C1-C8, en particulier méthanol, éthanol, iso-propanol, n-propanol, iso-butanol, tert-butanol, n-butanol, n-pentanol, sec-pentanol, cyclopentanol, n-hexanol, sec-hexanol, cyclohexanol, n-heptanol, sec-heptanol, n-octanol, sec-octanol.

6. Procédé selon l'une quelconque des revendications précédentes, **caractérisé en ce que** le solvant de lavage est différent du premier et/ou du deuxième solvant de cristallisation ou **en ce que** le solvant de lavage et le premier et/ou le deuxième solvant de cristallisation sont les mêmes.

7. Procédé selon l'une quelconque des revendications précédentes, **caractérisé en ce que** le lavage est effectué par lavage par déplacement et/ou par lavage par remise en suspension/suspension et/ou **en ce qu'**à l'étape (a) la charge est chauffée à une température dans la plage de 25 à 80 °C, en particulier dans la plage de 35 à 60 °C, et/ou **en ce qu'**à l'étape (b) la solution est refroidie à une température dans la plage de 20 à 35 °C et/ou **en ce qu'**à l'étape (c) l'ensemencement est effectué à une température dans la plage de 20 à 35 °C et/ou **en ce qu'**à l'étape (d) la solution est refroidie à une vitesse de refroidissement de 1 à 15 K/h.

8. Procédé selon l'une quelconque des revendications précédentes, **caractérisé en ce qu'**à l'étape (d) la solution est refroidie de manière linéaire ou **en ce qu'**à l'étape (d) la solution est refroidie avec un profil de refroidissement linéaire par morceaux ou **en ce qu'**à l'étape (d) la solution est refroidie avec un profil de refroidissement parabolique avec des vitesses de refroidissement croissantes ou **en ce qu'**à l'étape (d) la solution est refroidie avec un profil de refroidissement dynamique afin de maintenir une sursaturation relativement constante dans le cristallisoir ou **en ce qu'**à l'étape (d) la solution est refroidie par un procédé de refroidissement sous vide.

9. Procédé selon l'une quelconque des revendications précédentes, **caractérisé en ce qu'**à l'étape (d) la séparation des cristaux est effectuée par filtration tout en maintenant la température de la solution dans ± 5 °C de la température à laquelle la solution est refroidie à l'étape (d) et/ou **en ce que** le lavage du diéthylamino hydroxybenzoyl hexyl benzoate (I) cristallin séparé est effectué avec un solvant de lavage ayant une température dans ± 5 °C de la température à laquelle la solution est refroidie à l'étape (d) et/ou **en ce que** la cristallisation est effectuée dans un cristallisoir choisi parmi une cuve agitée avec chicanes, un cristallisoir à circulation forcée, un cristallisoir à tube de tirage, un cristallisoir à tube de tirage à chicanes, et un cristallisoir de type Oslo.

10. Procédé selon l'une quelconque des revendications précédentes, **caractérisé en ce que** le procédé comprend en outre les étapes suivantes :
i. dissoudre le diéthylamino hydroxybenzoyl hexyl benzoate (I) cristallin obtenu à l'étape (d) ou à l'étape (e) dans un solvant de purification ;
ii. mettre en contact la solution obtenue à l'étape (i) avec un adsorbant ; et
iii. éliminer le solvant de la solution obtenue à l'étape (ii) afin d'obtenir du diéthylamino hydroxybenzoyl hexyl benzoate (I) purifié.

11. Procédé selon la revendication 10, **caractérisé en ce que** le solvant de purification est choisi parmi le toluène, le cyclohexane et leurs combinaisons et/ou l'adsorbant est choisi parmi la silice, le charbon et leurs combinaisons.

12. Diéthylamino hydroxybenzoyl hexyl benzoate (I) cristallin obtenu par le procédé selon l'une quelconque des revendications 1 à 9, présentant
i) une teneur en ester dihexylique de l'acide phtalique (PSDHE) 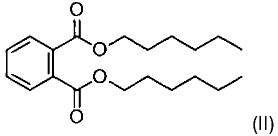 inférieure à 250 ppm ;
ii) une teneur en rhodamine 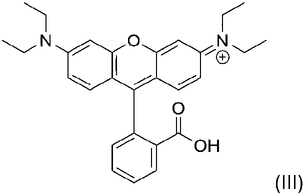 inférieure à 150 ppm ; et
iii) une teneur en ester hexylique de rhodamine 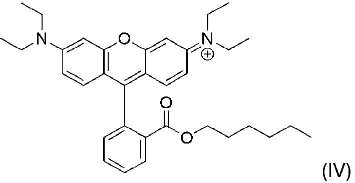 inférieure à 150 ppm ;
chacune sur la base du poids du diéthylamino hydroxybenzoyl hexyl benzoate (I) cristallin.

13. Diéthylamino hydroxybenzoyl hexyl benzoate (I) cristallin selon la revendication 12, **caractérisé en ce**
**que** la teneur en ester dihexylique de l'acide phtalique (PSDHE) 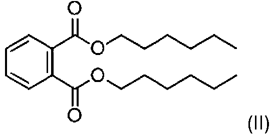 est inférieure à 1 ppm et/ou
la teneur en rhodamine 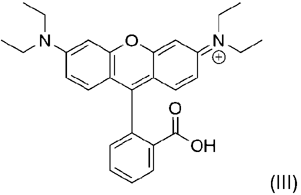 est inférieure à 10 ppm et/ou
la teneur en ester hexylique de rhodamine 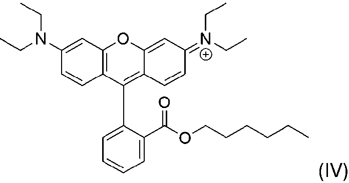 est inférieure à 10 ppm, chacune sur la base du poids du diéthylamino hydroxybenzoyl hexyl benzoate (I) cristallin.

14. Diéthylamino hydroxybenzoyl hexyl benzoate (I) purifié obtenu par le procédé selon la revendication 10 ou 11, présentant
i) une teneur en ester dihexylique de l'acide phtalique (PSDHE) 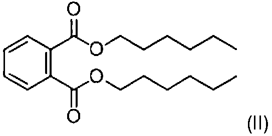 inférieure à 250 ppm ;
ii) une teneur en rhodamine 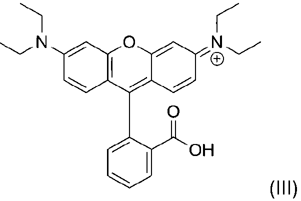 inférieure à 10 ppm ; et
iii) une teneur en ester hexylique de rhodamine 10 ppm ; 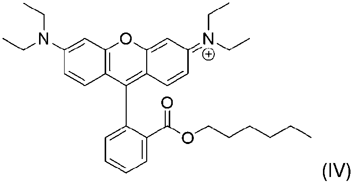 inférieure à
chacune sur la base du poids du diéthylamino hydroxybenzoyl hexyl benzoate (I) purifié.

15. Composition cosmétique comprenant le diéthylamino hydroxybenzoyl hexyl benzoate (I) purifié selon la revendication 14.
